# EUROPEAN PATENT SPECIFICATION

(11) **EP 2 553 091 B1**
(45) Date of publication and mention of the grant of the patent: **08.05.2019**
(21) Application number: 11709972.1
(22) Date of filing: 25.03.2011
(51) Int. Cl.: C12N 9/16, C12N 15/82, C12N 15/55, A01H 5/00

(54) **THERMOSTABLE PHYTASE VARIANTS**
THERMOSTABILE PHYTASEVARIANTEN
VARIANTS THERMOSTABLES D'UNE PHYTASE

(30) Priority: 26.03.2010 EP 10158031
(43) Date of publication of application: 06.02.2013
(73) Proprietor: Novozymes A/S, 2880 Bagsvaerd (DK)
(72) Inventor: DE MARIA, Leonardo, DK-2000 Frederiksberg (DK); SKOV, Lars Kobberoee, DK-Ballerup 2750 (DK); SKJOET, Michael, DK-4000 Roskilde (DK)
(74) Representative: NZ EPO Representatives
(86) International application number: PCT/EP2011/054652
(87) International publication number: WO 2011/117406

(56) References cited:
- WO-A2-2008/036916
- WO-A2-2008/055625
- US-A1- 2005 246 780
- MOON-SOO KIM ET AL: "Assembly of mutations for improving thermostability of Escherichia coli AppA2 phytase", APPLIED MICROBIOLOGY AND BIOTECHNOLOGY, SPRINGER, BERLIN, DE, vol. 79, no. 5, 29 April 2008 (2008-04-29) , pages 751-758, XP019623631, ISSN: 1432-0614
- LIM D ET AL: "CRYSTAL STRUCTURES OF ESCHERICHIA COLI PHYTASE AND ITS COMPLEX WITHPHYTATE", NATURE STRUCTURAL BIOLOGY, NATURE PUBLISHING GROUP, NEW YORK, NY, US, vol. 7, no. 2, 1 February 2000 (2000-02-01), pages 108-113, XP002938662, ISSN: 1072-8368, DOI: 10.1038/72371

### SEQUENCE LISTING

<110> Novozymes A/S
<120> THERMOSTABLE PHYTASE VARIANTS
<130> 11540-WO-PCT
<150> EP 10158031.4
   <151> 2010-03-26
<160> 6
<170> PatentIn version 3.5
<210> 1
   <211> 1239
   <212> DNA
   <213> Escherichia coli
<220>
   <221> CDS
   <222> (1)..(1239)
<400> 1
   cag agt gag ccg gag ctg aag ctg gaa agt gtg gtg att gtc agt cgt        48
   Gln Ser Glu Pro Glu Leu Lys Leu Glu Ser Val Val Ile Val Ser Arg
   1               5                   10                  15
   cat ggt gtg cgt gct cca acc aag gcc acg caa ctg atg cag gat gtc        96
   His Gly Val Arg Ala Pro Thr Lys Ala Thr Gln Leu Met Gln Asp Val
               20                  25                  30
   acc cca gac gca tgg cca acc tgg ccg gta aaa ctg ggt tgg ctg aca       144
   Thr Pro Asp Ala Trp Pro Thr Trp Pro Val Lys Leu Gly Trp Leu Thr
           35                  40                  45
   ccg cgc ggt ggt gag cta atc gcc tat ctc gga cat tac caa cgc cag       192
   Pro Arg Gly Gly Glu Leu Ile Ala Tyr Leu Gly His Tyr Gln Arg Gln
       50                  55                  60
   cgt ctg gta gcc gac gga ttg ctg gcg aaa aag ggc tgc ccg cag tct       240
   Arg Leu Val Ala Asp Gly Leu Leu Ala Lys Lys Gly Cys Pro Gln Ser
   65                  70                  75                  80
   ggt cag gtc gcg att att gct gat gtc gac gag cgt acc cgt aaa aca       288
   Gly Gln Val Ala Ile Ile Ala Asp Val Asp Glu Arg Thr Arg Lys Thr
                   85                  90                  95
   ggc gaa gcc ttc gcc gcc ggg ctg gca cct gac tgt gca ata acc gta       336
   Gly Glu Ala Phe Ala Ala Gly Leu Ala Pro Asp Cys Ala Ile Thr Val
               100                 105                 110
   cat acc cag aca gat acg tcc agt ccc gat ccg tta ttt aat cct cta       384
   His Thr Gln Thr Asp Thr Ser Ser Pro Asp Pro Leu Phe Asn Pro Leu
           115                 120                 125
   aaa act ggc gtt tgc caa ctg gat aac gcg aac gtg act gac gcg atc       432
   Lys Thr Gly Val Cys Gln Leu Asp Asn Ala Asn Val Thr Asp Ala Ile
       130                 135                 140
   ctc agc agg gca gga ggg tca att gct gac ttt acc ggg cat cgg caa       480
   Leu Ser Arg Ala Gly Gly Ser Ile Ala Asp Phe Thr Gly His Arg Gln
   145                 150                 155                 160
   acg gcg ttt cgc gaa ctg gaa cgg gtg ctt aat ttt ccg caa tca aac        528
   Thr Ala Phe Arg Glu Leu Glu Arg Val Leu Asn Phe Pro Gln Ser Asn
                   165                 170                 175
   ttg tgc ctt aaa cgt gag aaa cag gac gaa agc tgt tca tta acg cag        576
   Leu Cys Leu Lys Arg Glu Lys Gln Asp Glu Ser Cys Ser Leu Thr Gln
               180                 185                 190
   gca tta cca tcg gaa ctc aag gtg agc gcc gac aat gtc tca tta acc        624
   Ala Leu Pro Ser Glu Leu Lys Val Ser Ala Asp Asn Val Ser Leu Thr
           195                 200                 205
   ggt gcg gta agc ctc gca tca atg ctg acg gag ata ttt ctc ctg caa        672
   Gly Ala Val Ser Leu Ala Ser Met Leu Thr Glu Ile Phe Leu Leu Gln
       210                 215                 220
   caa gca cag gga atg ccg gag ccg ggg tgg gga agg atc acc gat tca        720
   Gln Ala Gln Gly Met Pro Glu Pro Gly Trp Gly Arg Ile Thr Asp Ser
   225                 230                 235                 240
   cac cag tgg aac acc ttg cta agt ttg cat aac gcg caa ttt tat ttg        768
   His Gln Trp Asn Thr Leu Leu Ser Leu His Asn Ala Gln Phe Tyr Leu
                   245                 250                 255
   cta caa cgc acg cca gag gtt gcc cgc agc cgc gcc acc ccg tta tta        816
   Leu Gln Arg Thr Pro Glu Val Ala Arg Ser Arg Ala Thr Pro Leu Leu
               260                 265                 270
   gat ttg atc aag aca gcg ttg acg ccc cat cca ccg caa aaa cag gcg        864
   Asp Leu Ile Lys Thr Ala Leu Thr Pro His Pro Pro Gln Lys Gln Ala
           275                 280                 285
   tat ggt gtg aca tta ccc act tca gtg ctg ttt atc gcc gga cac gat        912
   Tyr Gly Val Thr Leu Pro Thr Ser Val Leu Phe Ile Ala Gly His Asp
       290                 295                 300
   act aat ctg gca aat ctc ggc ggc gca ctg gag ctc aac tgg acg ctt        960
   Thr Asn Leu Ala Asn Leu Gly Gly Ala Leu Glu Leu Asn Trp Thr Leu
   305                 310                 315                 320
   ccc ggt cag ccg gat aac acg ccg cca ggt ggt gaa ctg gtg ttt gaa       1008
   Pro Gly Gln Pro Asp Asn Thr Pro Pro Gly Gly Glu Leu Val Phe Glu
                   325                 330                 335
   cgc tgg cgt cgg cta agc gat aac agc cag tgg att cag gtt tcg ctg       1056
   Arg Trp Arg Arg Leu Ser Asp Asn Ser Gln Trp Ile Gln Val Ser Leu
               340                 345                 350
   gtc ttc cag act tta cag cag atg cgt gat aaa acg ccg ctg tca tta       1104
   Val Phe Gln Thr Leu Gln Gln Met Arg Asp Lys Thr Pro Leu Ser Leu
           355                 360                 365
   aat acg ccg ccc gga gag gtg aaa ctg acc ctg gca gga tgt gaa gag       1152
   Asn Thr Pro Pro Gly Glu Val Lys Leu Thr Leu Ala Gly Cys Glu Glu
       370                 375                 380
   cga aat gcg cag ggc atg tgt tcg ttg gca ggt ttt acg caa atc gtg       1200
   Arg Asn Ala Gln Gly Met Cys Ser Leu Ala Gly Phe Thr Gln Ile Val
   385                 390                 395                 400
   aat gaa gca cgc ata ccg gcg tgc agt ttg cgt agt taa                   1239
   Asn Glu Ala Arg Ile Pro Ala Cys Ser Leu Arg Ser
                   405                 410
<210> 2
   <211> 412
   <212> PRT
   <213> Escherichia coli
<400> 2
   Gln Ser Glu Pro Glu Leu Lys Leu Glu Ser Val Val Ile Val Ser Arg
   1               5                   10                  15
   His Gly Val Arg Ala Pro Thr Lys Ala Thr Gln Leu Met Gln Asp Val
               20                  25                  30
   Thr Pro Asp Ala Trp Pro Thr Trp Pro Val Lys Leu Gly Trp Leu Thr
           35                  40                  45
   Pro Arg Gly Gly Glu Leu Ile Ala Tyr Leu Gly His Tyr Gln Arg Gln
       50                  55                  60
   Arg Leu Val Ala Asp Gly Leu Leu Ala Lys Lys Gly Cys Pro Gln Ser
   65                  70                  75                  80
   Gly Gln Val Ala Ile Ile Ala Asp Val Asp Glu Arg Thr Arg Lys Thr
                   85                  90                  95
   Gly Glu Ala Phe Ala Ala Gly Leu Ala Pro Asp Cys Ala Ile Thr Val
               100                 105                 110
   His Thr Gln Thr Asp Thr Ser Ser Pro Asp Pro Leu Phe Asn Pro Leu
           115                 120                 125
   Lys Thr Gly Val Cys Gln Leu Asp Asn Ala Asn Val Thr Asp Ala Ile
       130                 135                 140
   Leu Ser Arg Ala Gly Gly Ser Ile Ala Asp Phe Thr Gly His Arg Gln
   145                 150                 155                 160
   Thr Ala Phe Arg Glu Leu Glu Arg Val Leu Asn Phe Pro Gln Ser Asn
                   165                 170                 175
   Leu Cys Leu Lys Arg Glu Lys Gln Asp Glu Ser Cys Ser Leu Thr Gln
               180                 185                 190
   Ala Leu Pro Ser Glu Leu Lys Val Ser Ala Asp Asn Val Ser Leu Thr
           195                 200                 205
   Gly Ala Val Ser Leu Ala Ser Met Leu Thr Glu Ile Phe Leu Leu Gln
       210                 215                 220
   Gln Ala Gln Gly Met Pro Glu Pro Gly Trp Gly Arg Ile Thr Asp Ser
   225 230 235 240
   His Gln Trp Asn Thr Leu Leu Ser Leu His Asn Ala Gln Phe Tyr Leu
   245 250 255
   Leu Gln Arg Thr Pro Glu Val Ala Arg Ser Arg Ala Thr Pro Leu Leu
   260 265 270
   Asp Leu Ile Lys Thr Ala Leu Thr Pro His Pro Pro Gln Lys Gln Ala
   275 280 285
   Tyr Gly Val Thr Leu Pro Thr Ser Val Leu Phe Ile Ala Gly His Asp
   290 295 300
   Thr Asn Leu Ala Asn Leu Gly Gly Ala Leu Glu Leu Asn Trp Thr Leu
   305 310 315 320
   Pro Gly Gln Pro Asp Asn Thr Pro Pro Gly Gly Glu Leu Val Phe Glu
   325 330 335
   Arg Trp Arg Arg Leu Ser Asp Asn Ser Gln Trp Ile Gln Val Ser Leu
   340 345 350
   Val Phe Gln Thr Leu Gln Gln Met Arg Asp Lys Thr Pro Leu Ser Leu
   355 360 365
   Asn Thr Pro Pro Gly Glu Val Lys Leu Thr Leu Ala Gly Cys Glu Glu
   370 375 380
   Arg Asn Ala Gln Gly Met Cys Ser Leu Ala Gly Phe Thr Gln Ile Val
   385 390 395 400
   Asn Glu Ala Arg Ile Pro Ala Cys Ser Leu Arg Ser
   405 410
<210> 3
   <211> 412
   <212> PRT
   <213> Escherichia coli
<220>
   <221> mat_peptide
   <222> (1)..(412)
<400> 3
   Met Ala Gln Ser Glu Pro Glu Leu Lys Leu Glu Ser Val Val Ile Val
   1               5                   10                  15
   Ser Arg His Gly Val Arg Ala Pro Thr Lys Ala Thr Gln Leu Met Gln
               20                  25                  30
   Asp Val Thr Pro Asp Ala Trp Pro Thr Trp Pro Val Lys Leu Gly Trp
           35                  40                  45
   Leu Thr Pro Arg Gly Gly Glu Leu Ile Ala Tyr Leu Gly His Tyr Gln
       50                  55                  60
   Arg Gln Arg Leu Val Ala Asp Gly Leu Leu Ala Lys Lys Gly Cys Pro
   65                  70                  75                  80
   Gln Ser Gly Gln Val Ala Ile Ile Ala Asp Val Asp Glu Arg Thr Arg
                   85                  90                  95
   Lys Thr Gly Glu Ala Phe Ala Ala Gly Leu Ala Pro Asp Cys Ala Ile
               100                 105                 110
   Thr Val His Thr Gln Ala Asp Thr Ser Ser Pro Asp Pro Leu Phe Asn
           115                 120                 125
   Pro Leu Lys Thr Gly Val Cys Gln Leu Asp Asn Ala Asn Val Thr Asp
       130                 135                 140
   Ala Ile Leu Ser Arg Ala Gly Gly Ser Ile Ala Asp Phe Thr Gly His
   145                 150                 155                 160
   Arg Gln Thr Ala Phe Arg Glu Leu Glu Arg Val Leu Asn Phe Pro Gln
                   165                 170                 175
   Ser Asn Leu Cys Leu Lys Arg Glu Lys Gln Asp Glu Ser Cys Ser Leu
               180                 185                 190
   Thr Gln Ala Leu Pro Ser Glu Leu Lys Val Ser Ala Asp Asn Val Ser
           195                 200                 205
   Leu Thr Gly Ala Val Ser Leu Ala Ser Met Leu Thr Glu Ile Phe Leu
       210                 215                 220
   Leu Gln Gln Ala Gln Gly Met Pro Glu Pro Gly Trp Gly Arg Ile Thr
   225                 230                 235                 240
   Asp Ser His Gln Trp Asn Thr Leu Leu Ser Leu His Asn Ala Gln Phe
                   245                 250                 255
   Tyr Leu Leu Gln Arg Thr Pro Glu Val Ala Arg Ser Arg Ala Thr Pro
               260                 265                 270
   Leu Leu Asp Leu Ile Lys Thr Ala Leu Thr Pro His Pro Pro Gln Lys
           275                 280                 285
   Gln Ala Tyr Gly Val Thr Leu Pro Thr Ser Val Leu Phe Ile Ala Gly
       290                 295                 300
   His Asp Thr Asn Leu Ala Asn Leu Gly Gly Ala Leu Glu Leu Asn Trp
   305                 310                 315                 320
   Thr Leu Pro Gly Gln Pro Asp Asn Thr Pro Pro Gly Gly Glu Leu Val
                   325                 330                 335
   Phe Glu Arg Trp Arg Arg Leu Ser Asp Asn Ser Gln Trp Ile Gln Val
               340                 345                 350
   Ser Leu Val Phe Gln Thr Leu Gln Gln Met Arg Asp Lys Thr Pro Leu
           355                 360                 365
   Ser Leu Asn Thr Pro Pro Gly Glu Val Lys Leu Thr Leu Ala Gly Cys
       370                 375                 380
   Glu Glu Arg Asn Ala Gln Gly Met Cys Ser Leu Ala Gly Phe Thr Gln
   385                 390                 395                 400
   Ile Val Asn Glu Ala Arg Ile Pro Ala Cys Ser Leu
                   405                 410
<210> 4
   <211> 412
   <212> PRT
   <213> Escherichia coli
<220>
   <221> VARIANT
   <222> (1)..(412)
   <223> Variant Nov9X from WO 2009/073399
<400> 4
   Met Ala Gln Ser Glu Pro Glu Leu Lys Leu Glu Ser Val Val Ile Val
   1               5                   10                  15
   Ser Arg His Gly Val Arg Ala Pro Thr Lys Ala Thr Gln Leu Met Gln
               20                  25                  30
   Asp Val Thr Pro Asp Ala Trp Pro Thr Trp Pro Val Lys Leu Gly Glu
           35                  40                  45
   Leu Thr Pro Arg Gly Gly Glu Leu Ile Ala Tyr Leu Gly His Tyr Trp
       50                  55                  60
   Arg Gln Arg Leu Val Ala Asp Gly Leu Leu Pro Lys Cys Gly Cys Pro
   65                  70                  75                  80
   Gln Ser Gly Gln Val Ala Ile Ile Ala Asp Val Asp Glu Arg Thr Arg
                   85                  90                  95
   Lys Thr Gly Glu Ala Phe Ala Ala Gly Leu Ala Pro Asp Cys Ala Ile
               100                 105                 110
   Thr Val His Thr Gln Ala Asp Thr Ser Ser Pro Asp Pro Leu Phe Asn
           115                 120                 125
   Pro Leu Lys Thr Gly Val Cys Gln Leu Asp Asn Ala Asn Val Thr Asp
       130                 135                 140
   Ala Ile Leu Glu Arg Ala Gly Gly Ser Ile Ala Asp Phe Thr Gly His
   145                 150                 155                 160
   Tyr Gln Thr Ala Phe Arg Glu Leu Glu Arg Val Leu Asn Phe Pro Gln
                   165                 170                 175
   Ser Asn Leu Cys Leu Lys Arg Glu Lys Gln Asp Glu Ser Cys Ser Leu
               180                 185                 190
   Thr Gln Ala Leu Pro Ser Glu Leu Lys Val Ser Ala Asp Cys Val Ser
           195                 200                 205
   Leu Thr Gly Ala Val Ser Leu Ala Ser Met Leu Thr Glu Ile Phe Leu
       210                 215                 220
   Leu Gln Gln Ala Gln Gly Met Pro Glu Pro Gly Trp Gly Arg Ile Thr
   225                 230                 235                 240
   Asp Ser His Gln Trp Asn Thr Leu Leu Ser Leu His Asn Ala Gln Phe
                   245                 250                 255
   Asp Leu Leu Gln Arg Thr Pro Glu Val Ala Arg Ser Arg Ala Thr Pro
               260                 265                 270
   Leu Leu Asp Leu Ile Lys Thr Ala Leu Thr Pro His Pro Pro Gln Lys
           275                 280                 285
   Gln Ala Tyr Gly Val Thr Leu Pro Thr Ser Val Leu Phe Ile Ala Gly
       290                 295                 300
   His Asp Thr Asn Leu Ala Asn Leu Gly Gly Ala Leu Glu Leu Asn Trp
   305                 310                 315                 320
   Thr Leu Pro Gly Gln Pro Asp Asn Thr Pro Pro Gly Gly Glu Leu Val
                   325                 330                 335
   Phe Glu Arg Trp Arg Arg Leu Ser Asp Asn Ser Gln Trp Ile Gln Val
   340 345 350
   Ser Leu Val Phe Gln Thr Leu Gln Gln Met Arg Asp Lys Thr Pro Leu
   355 360 365
   Ser Leu Asn Thr Pro Pro Gly Glu Val Lys Leu Thr Leu Ala Gly Cys
   370 375 380
   Glu Glu Arg Asn Ala Gln Gly Met Cys Ser Leu Ala Gly Phe Thr Gln
   385 390 395 400
   Ile Val Asn Glu Ala Arg Ile Pro Ala Cys Ser Leu
   405 410
<210> 5
   <211> 432
   <212> PRT
   <213> Escherichia coli
<220>
   <221> PROPEP
   <222> (1)..(22)
<220>
   <221> mat_peptide
   <222> (23)..(432)
<400> 5
   Met Lys Ala Ile Leu Ile Pro Phe Leu Ser Leu Leu Ile Pro Leu Thr
           -20                 -15                 -10
   Pro Gln Ser Ala Phe Ala Gln Ser Glu Pro Glu Leu Lys Leu Glu Ser
       -5                  -1  1               5                   10
   Val Val Ile Val Ser Arg His Gly Val Arg Ala Pro Thr Lys Ala Thr
                   15                  20                  25
   Gln Leu Met Gln Asp Val Thr Pro Asp Ala Trp Pro Thr Trp Pro Val
               30                  35                  40
   Lys Leu Gly Glu Leu Thr Pro Arg Gly Gly Glu Leu Ile Ala Tyr Leu
           45                  50                  55
   Gly His Tyr Trp Arg Gln Arg Leu Val Ala Asp Gly Leu Leu Pro Lys
       60                  65                  70
   Cys Gly Cys Pro Gln Ser Gly Gln Val Ala Ile Ile Ala Asp Val Asp
   75                  80                  85                  90
   Glu Arg Thr Arg Lys Thr Gly Glu Ala Phe Ala Ala Gly Leu Ala Pro
                   95                  100                 105
   Asp Cys Ala Ile Thr Val His Thr Gln Ala Asp Thr Ser Ser Pro Asp
               110                 115                 120
   Pro Leu Phe Asn Pro Leu Lys Thr Gly Val Cys Gln Leu Asp Asn Ala
           125                 130                 135
   Asn Val Thr Asp Ala Ile Leu Glu Arg Ala Gly Gly Ser Ile Ala Asp
       140                 145                 150
   Phe Thr Gly His Tyr Gln Thr Ala Phe Arg Glu Leu Glu Arg Val Leu
   155                 160                 165                 170
   Asn Phe Pro Gln Ser Asn Leu Cys Leu Lys Arg Glu Lys Gln Asp Glu
                   175                 180                 185
   Ser Cys Ser Leu Thr Gln Ala Leu Pro Ser Glu Leu Lys Val Ser Ala
               190                 195                 200
   Asp Cys Val Ser Leu Thr Gly Ala Val Ser Leu Ala Ser Met Leu Thr
           205                 210                 215
   Glu Ile Phe Leu Leu Gln Gln Ala Gln Gly Met Pro Glu Pro Gly Trp
       220                 225                 230
   Gly Arg Ile Thr Asp Ser His Gln Trp Asn Thr Leu Leu Ser Leu His
   235                 240                 245                 250
   Asn Ala Gln Phe Asp Leu Leu Gln Arg Thr Pro Glu Val Ala Arg Ser
                   255                 260                 265
   Arg Ala Thr Pro Leu Leu Asp Leu Ile Lys Thr Ala Leu Thr Pro His
               270                 275                 280
   Pro Pro Gln Lys Gln Ala Tyr Gly Val Thr Leu Pro Thr Ser Val Leu
           285                 290                 295
   Phe Ile Ala Gly His Asp Thr Asn Leu Ala Asn Leu Gly Gly Ala Leu
       300                 305                 310
   Glu Leu Asn Trp Thr Leu Pro Gly Gln Pro Asp Asn Thr Pro Pro Gly
   315                 320                 325                 330
   Gly Glu Leu Val Phe Glu Arg Trp Arg Arg Leu Ser Asp Asn Ser Gln
                   335                 340                 345
   Trp Ile Gln Val Ser Leu Val Phe Gln Thr Leu Gln Gln Met Arg Asp
               350                 355                 360
   Lys Thr Pro Leu Ser Leu Asn Thr Pro Pro Gly Glu Val Lys Leu Thr
   365 370 375
   Leu Ala Gly Cys Glu Glu Arg Asn Ala Gln Gly Met Cys Ser Leu Ala
   380 385 390
   Gly Phe Thr Gln Ile Val Asn Glu Ala Arg Ile Pro Ala Cys Ser Leu
   395 400 405 410
<210> 6
   <211> 432
   <212> PRT
   <213> Escherichia coli
<220>
   <221> PROPEP
   <222> (1)..(22)
<220>
   <221> mat_peptide
   <222> (23)..(432)
<400> 6
   Met Lys Ala Ile Leu Ile Pro Phe Leu Ser Leu Leu Ile Pro Leu Thr
   -20 -15 -10
   Pro Gln Ser Ala Phe Ala Gln Ser Glu Pro Glu Leu Lys Leu Glu Ser
   -5 -1 1 5 10
   Val Val Ile Val Ser Arg His Gly Val Arg Ala Pro Thr Lys Ala Thr
   15 20 25
   Gln Leu Met Gln Asp Val Thr Pro Asp Ala Trp Pro Thr Trp Pro Val
   30 35 40
   Lys Leu Gly Trp Leu Thr Pro Arg Gly Gly Glu Leu Ile Ala Tyr Leu
   45 50 55
   Gly His Tyr Gln Arg Gln Arg Leu Val Ala Asp Gly Leu Leu Ala Lys
   60 65 70
   Lys Gly Cys Pro Gln Ser Gly Gln Val Ala Ile Ile Ala Asp Val Asp
   75 80 85 90
   Glu Arg Thr Arg Lys Thr Gly Glu Ala Phe Ala Ala Gly Leu Ala Pro
   95 100 105
   Asp Cys Ala Ile Thr Val His Thr Gln Ala Asp Thr Ser Ser Pro Asp
   110 115 120
   Pro Leu Phe Asn Pro Leu Lys Thr Gly Val Cys Gln Leu Asp Asn Ala
           125                 130                 135
   Asn Val Thr Asp Ala Ile Leu Ser Arg Ala Gly Gly Ser Ile Ala Asp
       140                 145                 150
   Phe Thr Gly His Arg Gln Thr Ala Phe Arg Glu Leu Glu Arg Val Leu
   155                 160                 165                 170
   Asn Phe Pro Gln Ser Asn Leu Cys Leu Lys Arg Glu Lys Gln Asp Glu
                   175                 180                 185
   Ser Cys Ser Leu Thr Gln Ala Leu Pro Ser Glu Leu Lys Val Ser Ala
               190                 195                 200
   Asp Asn Val Ser Leu Thr Gly Ala Val Ser Leu Ala Ser Met Leu Thr
           205                 210                 215
   Glu Ile Phe Leu Leu Gln Gln Ala Gln Gly Met Pro Glu Pro Gly Trp
       220                 225                 230
   Gly Arg Ile Thr Asp Ser His Gln Trp Asn Thr Leu Leu Ser Leu His
   235                 240                 245                 250
   Asn Ala Gln Phe Tyr Leu Leu Gln Arg Thr Pro Glu Val Ala Arg Ser
                   255                 260                 265
   Arg Ala Thr Pro Leu Leu Asp Leu Ile Lys Thr Ala Leu Thr Pro His
               270                 275                 280
   Pro Pro Gln Lys Gln Ala Tyr Gly Val Thr Leu Pro Thr Ser Val Leu
           285                 290                 295
   Phe Ile Ala Gly His Asp Thr Asn Leu Ala Asn Leu Gly Gly Ala Leu
       300                 305                 310
   Glu Leu Asn Trp Thr Leu Pro Gly Gln Pro Asp Asn Thr Pro Pro Gly
   315                 320                 325                 330
   Gly Glu Leu Val Phe Glu Arg Trp Arg Arg Leu Ser Asp Asn Ser Gln
                   335                 340                 345
   Trp Ile Gln Val Ser Leu Val Phe Gln Thr Leu Gln Gln Met Arg Asp
               350                 355                 360
   Lys Thr Pro Leu Ser Leu Asn Thr Pro Pro Gly Glu Val Lys Leu Thr
           365                 370                 375
   Leu Ala Gly Cys Glu Glu Arg Asn Ala Gln Gly Met Cys Ser Leu Ala
       380                 385                 390
   Gly Phe Thr Gln Ile Val Asn Glu Ala Arg Ile Pro Ala Cys Ser Leu
   395                 400                 405                 410

## Description

### Field of the Invention

The present disclosure relates to a method of producing a variant phytase having at least 70% identity to SEQ ID NO:2 and comprising the establishment of at least one disulfide bridge as compared to SEQ ID NO:2, wherein said at least one disulfide bridge is not among the four naturally occurring ones in positions 77/108, 133/408, 178/188, and 382/391 with the numbering as provided in SEQ ID NO:2 and closely related phytases (i.e., is a variant thereof). The disclosure also relates to DNA encoding these phytases, methods of their production, as well as the use thereof, e.g. in animal feed and animal feed additives. The mature part of the Escherichia coli phytase reference is included in the sequence listing as SEQ ID NO:2. The reference phytase is the one deposited in UNIPROT under the id. A7ZK83.

### Background of the Invention

### Background art

Phytases are well-known enzymes, as are the advantages of adding them to foodstuffs for animals, including humans. Phytases have been isolated from various sources, including a number of fungal and bacterial strains.

It is an object of the present invention to provide alternative polypeptides having phytase activity (phytases) and polynucleotides encoding the polypeptides. The phytase variants of the disclosure exhibit modified or altered preferably improved properties as compared to the parent phytase. Non-limiting examples of such properties are: Stability (such as acid-stability, heat-stability, steam stability, pelleting stability, and/or protease stability, in particular pepsin stability), temperature profile, pH profile, specific activity, substrate specificity, performance in animal feed(such as an improved release and/or degradation of phytate), susceptibility to glycation, and/or glycosylation pattern.

As described herein, mutagenesis of a parent polynucleotide encoding a phytase is employed to prepare variant (synthetic) DNAs encoding a phytase having improved properties relative to the phytase encoded by the parent polynucleotide.

A number of three-dimensional structures of phytases of the Histidine acid phosphate (HAP) type are known. (e.g. Lim et al. Nature struct. biol. 7, 108-113 (2000)). From these it has been found that they all have four disulfide bridges located at the position pairs 77/108 133/407 178/187 381/390 (according to the numbering used here). Typically these occupy all the cysteines present in the molecule.

### Escherichia

The sequence of the appA gene from E. coli was published in 1990 by Dassa et al. (J. Bact. P5497-5500, 1990). The E. coli phytase comprises the four disulfide bridges indicated above and no further cysteines are present.

Variants of the E. coli phytase were first disclosed in Ostanin et al. 1992 (J. Biol. Chem. 267(32) November 15, pp 22830-22836, 1992) [R16A, H17N, R20A, R92A, H303A, R63A] showing that the replacement of the R16 and H17 was detrimental to the activity and also that the replacement of the R20, R92 and H303 reduced the activity very strongly, whereas the R63 replacement provided a minor positive effect on activity.

Other variants of the E. coli phytase have been disclosed in WO 2006/028684 (Diversa Corp.), WO 2009/073399 (Syngenta Part. AG), WO 2008/036916 (Verenium Inc.), WO 02/095003 (Diversa Corp.),

It is an object of the disclosure to provide phytases of modified, preferably, improved temperature properties. Non-limiting examples of such properties are: Thermostability, and temperature profile.

### Brief Description of the Drawings and Sequence listing

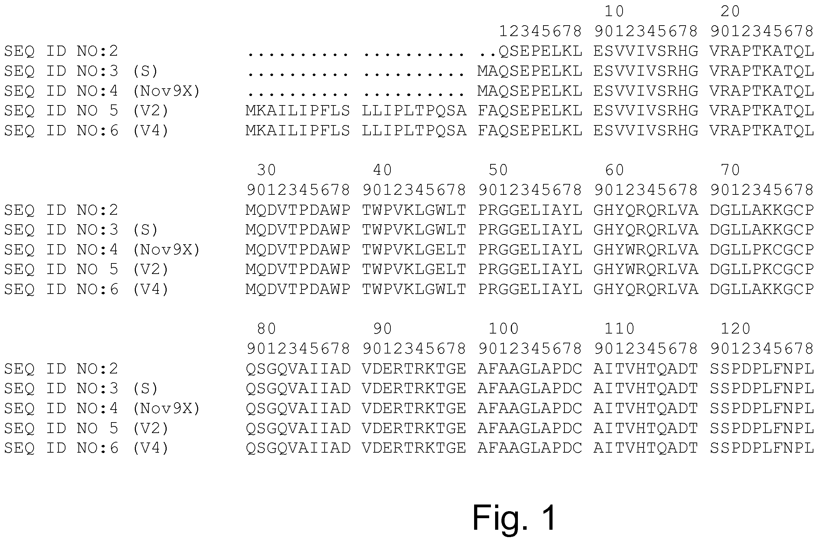

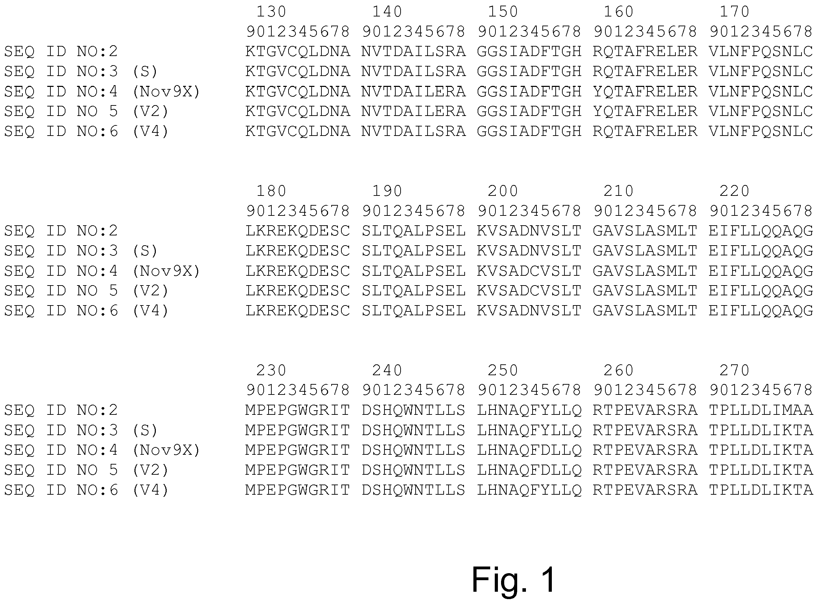

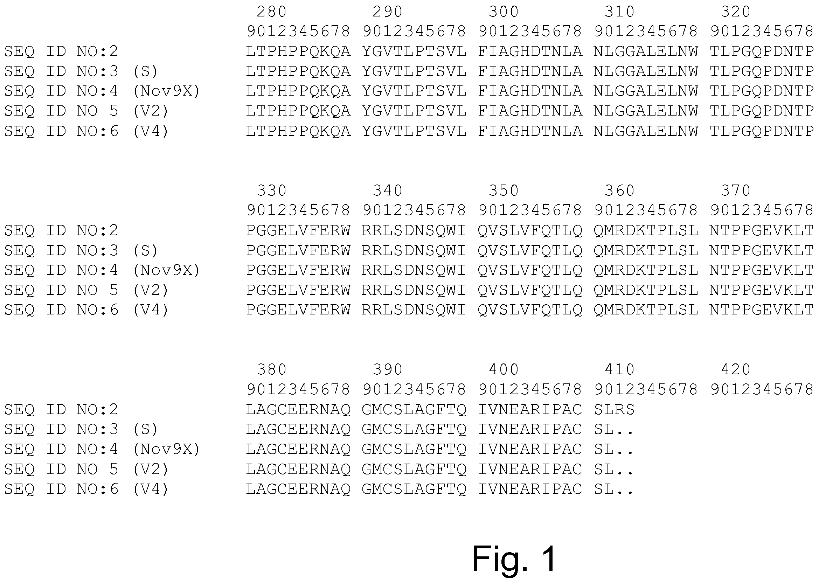

Fig. 1 is an alignment of the phytases of SEQ ID NOs:2 to 6. The position numbers in Fig. 1 refer to the numbering of SEQ ID NO:2.

In the Figure (Sequence listing) the sequences apply as follows:

| | |
|---|---|
| SEQ ID NO:1 | DNA encoding SEQ ID NO:2 |
| SEQ ID NO:2 | E. coli phytase wild type |
| SEQ ID NO:3 | Wild type from WO 2009/073399 |
| SEQ ID NO:4 | Variant Nov9X from WO 2009/073399 |
| SEQ ID NO:5 | SEQ ID NO:2 from WO 2008/036916 (18 and 323 are Xaa) |
| SEQ ID NO:6 | SEQ ID NO:4 from WO 2008/036916 |

### Summary of Examples

In the specification the following examples are provided:
Example 1: Preparation of variants, and determination of activity
Example 2: Specific activity
Example 3. Thermostability
Example 4. Temperature profile
Example 5. pH profile
Example 6: Performance in animal feed in an in vitro model
Example 7: Performance in an in vivo pig trial

### Detailed Description of the Invention

In a first aspect, the present invention relates to a phytase variant having at least 85% identity to SEQ ID NO:2 and comprising the establishment of at least one disulfide bridge as compared to SEQ ID NO:2, wherein said at least one disulfide bridge is not among the four naturally occurring ones in positions 77/108, 133/408, 178/188, and 382/391 with the numbering as provided in SEQ ID NO:2, wherein the at least one disulfide bridge is position pair: A) 52C/99C, and wherein the phytase variant is more thermostable than the reference phytase.

The percentage of identity is determined as described in the section "Phytase Polypeptides, Percentage of Identity".

The position numbers refer to the position numbering of SEQ ID NO:2, as described in the section "Position Numbering." Positions corresponding to these SEQ ID NO:2 position numbers in other phytases are determined as described in the section "Identifying Corresponding Position Numbers."

The at least one disulfide bridge is position pair: A) 52C/99C..

According to the invention a first disulfide bridge is preferably established in the position pair A between the residues in positions 52 and 99 and a second disulfide bridge is established in the position pair B between the residues in positions 141 and 200.

In certain embodiments the number of established disulfide bridges is 2, 3, 4, 5, 6, and/or 7.

When the number of established disulfide bridges is two the following combinations of position pairs may be created: A+B, A+C, A+D, A+E, A+F and A+G,.

When the number of established disulfide bridges is three the following combinations of position pairs may be created:
A+B+C, A+B+D, A+B+E, A+B+F, A+B+G, A+C+D, A+C+E, A+C+F, A+C+G, A+D+E, A+D+F, A+D+G, A+E+F, A+E+G and A+F+G.

When the number of established disulfide bridges is four the following combinations of position pairs may be created: A+B+C+D, A+B+C+E, A+B+C+F, A+B+C+G, A+B+D+E, A+B+D+F, A+B+D+G, A+B+E+F, A+B+E+G, A+B+F+G, A+C+D+E, A+C+D+F, A+C+D+G, A+C+E+F, A+C+E+G, A+C+E+H, A+C+F+G, A+D+E+F, A+D+E+G, A+D+F+G and A+E+F+G.

When the number of established disulfide bridges is five the following combinations of position pairs may be created:'A+B+C+D+E, A+B+C+D+F, A+B+C+D+G, A+B+C+E+F, A+B+C+E+G, A+B+C+F+G, A+B+D+E+F, A+B+D+E+G, A+B+D+F+G, A+B+E+F+G, A+B+F+G+H, A+C+D+E+F, A+C+D+E+G, A+C+D+F+G, A+C+E+F+G, and A+D+E+F+G. When the number of established disulfide bridges is six the following combinations of position pairs may be created: A+B+C+D+E+F, A+B+C+D+E+G, A+B+C+D+F+G, A+B+C+E+F+G, A+B+D+E+F+G and A+C+D+E+F+G.When the number of established disulfide bridges is seven the following combination of position pairs may be created: A+B+C+D+E+F+G.

In all of the above combinations A means 52C/99C, B means 141C/200C, C means 31C/177C, D means 91C/46C, E means 31C/176C, F means 59C/100C, and G means 162C/248C.

According to the invention the phytase variant may further comprise at least one modification in at least one position selected from the following: 25, 37, 38, 75, 77, 108, 114, 123, 126, 127, 133, 137, 141, 142, 146, 157, 173, 178, 188, 204, 211, 233, 235, 253, 267, 286, 287, 317, 318, 327, 367, 382, 391, and 408.

The invention further provides that the above modifications specifically are chosen from the following modifications: 25F, 37F, 38Y, 75K, 75V, 75E, 77A, 108A, 114H, 123E, 126Y, 127L, 127V, 133A, 137E, 137V, 141R, 142R, 146R, 157R, 173Y, 178A, 188A, 204N, 204D, 211W, 233E, 235Y, 253V, 267A, 286F, 287Y, 317L, 318Y, 327Y, 367F, 382A, 391A, and 408A.

According to the invention, the additional disulfide bridges is G52C/A99C. In further specific embodiments of the invention, further specific modifications are selected from: A25F, W37F, P38Y, C75K, C75V, C75E, C77A, C108A, T114H, P123E, N126Y, P127L, P127V, C133A, N137E, N137V, T141R, D142R, E146R, G157R, P173Y, C178A, C188A, C204N, C204D, V211W, G233E, G235Y, Q253V, R267A, K286F, Q287Y, N317L, W318Y, T327Y, S367F, C382A, C391A, C408A,
and/or from the following combinations W37F/P38Y, P123E/P127L, N126Y/P127V, G233E/G235Y, K286F/Q287Y, N317L/W318Y, W37F/P38Y/P123E/P127L, W37F/P38Y/N126Y/P127V, P173Y/N317L/W318Y, C75K/C204N, C75K/C204N/C178A/C188A,C75K/C204N/C382A/C391A, C75K/C204N/C178A/C188A/C382A/C391A, C75K/C204N/C77A/C108A, C75K/C204N/C133A/C108A A25F/C75V/T114H/N137E/T141R/E146R/G157R/C204D/V211W/Q253V/R267A/T327Y C178A/C188A A25F/C75V/T114H/N137E/T141R/E146R/G157R/C204D/V211W/Q253V/R267A/T327Y/C382A/C391A A25F/C75V/T114H/N137E/T141R/E146R/G157R/C204D/V211W/Q253V/R267A/T327Y/C178A/C188A/C382A/C391A A25F/C75V/T114H/N137E/T141R/E146R/G157R/C204D/V211W/Q253V/R267A/T327Y/C77A/C108A A25F/C75V/T114H/N137E/T141R/E146R/G157R/C204D/V211W/Q253V/R267A/T327Y/C133A/C408A A25F/C75E/T114H/N137V/G157R/C204D/V211W/Q253V/R267A/T327Y A25F/C75E/N137V/T141R/D142R/G157R/C204D/V211W/Q253V/T327Y A25F/T114H/N137V/T141R/D142R/G157R/C204D/V211W/Q253V/T327Y A25F/T114H/N137V/T141R/D142R/E146R/G157R/C204DA/211W/Q253V/R267A/T327Y A25F/C75V/T114H/N137V/T141R/E146R/G157R/C204DA/211W/Q253V/R267A/T327Y A25F/C75V/T114H/N137V/T141R/D142R/E146R/G157R/C204D/V211W/Q253V/T327Y A25F/T114H/N137V/T141R/D142R/E146R/G157R/C204D/V211W/Q253V/T327Y A25F/C75E/T114H/N137V/T141R/D142R/G157R/C204D/V211W/Q253V/T327Y A25F/C75E/T114H/N137V/T141R/D142R/E146R/G157R/V211W/Q253V/R267A/T327Y A25F/C75E/T114H/N137E/T141R/D142R/G157R/V211W/Q253V/R267A/T327Y/L341P A47F/C97V/T136H/N159V/T163R/D164R/G179R/V233W/Q275V/R289A/T349YA25F/C75E/T114H/N137V/T141R/D142R/E146R/G157R/C204D/V211W/Q253V/R267A/T327YA25F/C75E/N137V/D142R/G157R/C204D/V/211W/Q253V/R267A/T327Y A25F/C75E/N137E/T141R/D142R/E146R/G157R/C204D/V211W/Q253V/R267A/T327Y A25F/C75V/T114H/N137E/T141R/D142R/E146R/G157R/C204D/V211W/Q253V/ R267A/T327Y A25F/C75E/T114H/N137V/T141R/G157R/C204D/V211W/Q253V/T327Y A25F/C75E/T114H/N137V/T141R/D142R/E146R/G157R/C204D/V211W/Q253V/T327Y A25F/T114H/N137E/T141R/D142R/G157R/C204D/V211W/Q253V/R267A/T327Y A25F/T114H/N137V/D142R/G157R/C204D/V/211W/Q253V/T327Y A25F/C75V/T114H/N137V/D142R/G157R/C204D/V211W/Q253V/T327Y A25F/C75V/T114H/N137V/D142R/G157R/V211W/Q253V/T327Y A25F/T114H/N137V/C204D/V211W/T327Y

In further aspect, the invention relates to a method of preparing the phytase variant of the invention, comprising the establishment of at least one disulfide bridge as compared to SEQ ID NO: 2, wherein said disulfide bridge is not among the four naturally occurring ones in positions 77/108, 133/408, 178/188, and 382/391 with the numbering as provided in SEQ ID NO: 2, and wherein the at least one disulfide bridge is established in position pair: A) 52C/99C.

The method of the invention may be used to create a variant of any wildtype or variant phytase. In particular embodiments, it produces a variant of the mature part of the phytase of of SEQ ID NO:2, SEQ ID NO:3, SEQ ID NO:4, SEQ ID NO:5, or SEQ ID NO:6 is used as a parent/backbone for producing the phytase variant.

The method of the invention provides a phytase wherein the phytase variant is more thermostable than the reference phytase. The method of the disclosure may provide a phytase variant having further improved properties, such as heat-stability, steam stability, temperature profile, pelleting stability, acid-stability, pH profile, and/or protease stability, in particular pepsin stability, specific activity, substrate specificity, performance in animal feed (such as an improved release and/or degradation of phytate), susceptibility to glycation, and/or glycosylation pattern. The variants provided by the invention exhibit especially improved thermal properties, such as thermostability, heat-stability, steam stability, temperature profile, pelleting stability or improved performance in animal feed.

The method of the invention thus relates to phytase variants having improved thermal properties, such as thermostability, heat-stability, steam stability, temperature profile, and/or pelleting stability.

The method of the invention thus relates to phytase variants having improved thermostability.

The method of the disclosure thus relates to phytase variants having improved heat-stability.

The method of the disclosure thus relates to phytase variants having improved steam stability.

The method of the disclosure thus relates to phytase variants having improved temperature profile.

The method of the disclosure thus relates to phytase variants having improved pelleting stability.

The method of the disclosure thus relates to phytase variants having improved acid-stability.

The method of the disclosure thus relates to phytase variants having improved pH profile.

The method of the disclosure thus relates to phytase variants having improved protease stability, in particular pepsin stability.

The method of the disclosure thus relates to phytase variants having improved specific activity.

The method of the disclosure thus relates to phytase variants having improved substrate specificity.

The method of the disclosure thus relates to phytase variants having improved performance in animal feed (such as an improved release and/or degradation of phytate).

The method of the disclosure thus relates to phytase variants having improved susceptibility to glycation.

The method of the disclosure thus relates to phytase variants having improved and/or glycosylation pattern.

The invention further relates to polynucleotide comprising nucleotide sequences which encode the phytase variants of the invention, nucleic acid constructs comprising the polynucleotides operably linked to one or more control sequences that direct the production of the polypeptide in an expression host, recombinant expression vectors comprising such nucleic acid constructs, and recombinant host cells comprising a nucleic acid construct and/or an expression vector.

The invention further relates to methods for producing phytase variants as provided comprising
(a) cultivating a host cell to produce a supernatant comprising the phytase; and
(b) recovering the phytase.

The disclosure further relates to transgenic plants, or plant part, capable of expressing the phytase variants.

The invention further relates to compositions comprising at least one phytase variant of the invention, and (a) at least one fat soluble vitamin; (b) at least one water soluble vitamin; and/or (c) at least one trace mineral. Such compositions may further comprise at least one enzyme selected from the following group of enzymes: amylase, phytase, phosphatase, xylanase, galactanase, alpha-galactosidase, protease, phospholipase, and/or beta-glucanase. The compositions may be animal feed additives that may have a crude protein content of 50 to 800 g/kg and comprising a phytase variant of the invention.

The invention further relates to methods for improving the nutritional value of an animal feed, by adding a phytase variant of the invention to the feed, processes for reducing phytate levels in animal manure by feeding an animal with an effective amount of the feed, methods for the treatment of vegetable proteins, comprising the step of adding a phytase variant to at least one vegetable protein, and the use of a phytase variant of a composition of the invention.

The invention also provides a method for producing a fermentation product such as, e.g., ethanol, beer, wine, comprising fermenting a carbohydrate material in the presence of a phytase variant, a method for producing ethanol comprising fermenting a carbohydrate material in the presence of a phytase variant and producing ethanol.

### Phytase Polypeptides, Percentage of Identity

In the present context a phytase is a polypeptide having phytase activity, i.e. an enzyme which catalyzes the hydrolysis of phytate (myo-inositol hexakisphosphate) to (1) myo-inositol and/or (2) mono-, di-, tri-, tetra- and/or penta-phosphates thereof and (3) inorganic phosphate.

In the present context the term a phytase substrate encompasses, i.a., phytic acid and any phytate (salt of phytic acid), as well as the phosphates listed under (2) above.

The ENZYME site at the internet (http://www.expasy.ch/enzyme/) is a repository of information relative to the nomenclature of enzymes. It is primarily based on the recommendations of the Nomenclature Committee of the International Union of Biochemistry and Molecular Biology (IUB-MB) and it describes each type of characterized enzyme for which an EC (Enzyme Commission) number has been provided (Bairoch A. The ENZYME database, 2000, Nucleic Acids Res 28:304-305). See also the handbook Enzyme Nomenclature from NC-IUBMB, 1992).

According to the ENZYME site, three different types of phytases are known: A so-called 3-phytase (alternative name 1-phytase; a myo-inositol hexaphosphate 3-phosphohydrolase, EC 3.1.3.8), a so-called 4-phytase (alternative name 6-phytase, name based on 1L-numbering system and not 1D-numbering, EC 3.1.3.26), and a so-called 5-phytase (EC 3.1.3.72). For the purposes of the present invention, all three types are included in the definition of phytase.

In a particular embodiment, the phytases of the invention belong to the family of acid histidine phosphatases, which includes the Escherichia coli pH 2.5 acid phosphatase (gene appA) as well as fungal phytases such as Aspergillus awamorii phytases A and B (EC: 3.1.3.8) (gene phyA and phyB). The histidine acid phosphatases share two regions of sequence similarity, each centered around a conserved histidine residue. These two histidines seem to be involved in the enzymes' catalytic mechanism. The first histidine is located in the N-terminal section and forms a phosphor-histidine intermediate while the second is located in the C-terminal section and possibly acts as proton donor.

In a further particular embodiment, the phytases of the invention have a conserved active site motif, viz. R-H-G-X-R-X-P, wherein X designates any amino acid (see amino acids 16 to 22 of SEQ ID NOs:2, 3, 4, 6 and amino acids 38-44 of SEQ ID NO:9). In a preferred embodiment, the conserved active site motif is R-H-G-V-R-A-P, i.e. amino acids 16-22 (by reference to SEQ ID NO:2) are RHGVRAP.

For the purposes of the present invention the phytase activity is determined in the unit of FYT, one FYT being the amount of enzyme that liberates 1 micro-mol inorganic ortho-phosphate per min. under the following conditions: pH 5.5; temperature 37°C; substrate: sodium phytate (C₆ H₆O₂₄P₆Na₁₂) in a concentration of 0.0050 mol/l. Suitable phytase assays are the FYT and FTU assays described in Example 1 of WO 00/20569. FTU is for determining phytase activity in feed and premix. Phytase activity may also be determined using the assays of Example 1 ("Determination of phosphatase activity" or "Determination of phytase activity").

In a particular embodiment the phytase of the invention is isolated. The term "isolated" as used herein refers to a polypeptide which is at least 20% pure, preferably at least 40% pure, more preferably at least 60% pure, even more preferably at least 80% pure, most preferably at least 90% pure, and even most preferably at least 95% pure, as determined by SDS-PAGE. In particular, it is preferred that the polypeptides are in "essentially pure form", i.e., that the polypeptide preparation is essentially free of other polypeptide material with which it is natively associated. This can be accomplished, for example, by preparing the polypeptide by means of well-known recombinant methods or by classical purification methods.

The relatedness between two amino acid sequences is described by the parameter "identity". For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence of the present invention ("invention sequence") and the amino acid sequence referred to in the claims (SEQ ID NO:2) is calculated as the number of exact matches in an alignment of the two sequences, divided by the length of the "invention sequence," or the length of the SEQ ID NO:2, whichever is the shortest. The result is expressed in percent identity.

An exact match occurs when the "invention sequence" and SEQ ID NO:2 have identical amino acid residues in the same positions of the overlap (in the alignment example below this is represented by "|"). The length of a sequence is the number of amino acid residues in the sequence (e.g. the length of amino acids 1-411 of SEQ ID NO:2 is 411).

In a purely hypothetical, alignment example below, the overlap is the amino acid sequence "HTWGER-NL" of Sequence 1; or the amino acid sequence "HGWGEDANL" of Sequence 2. In the example a gap is indicated by a "-".

Hypothetical alignment example:

In a particular embodiment, the percentage of identity of an amino acid sequence of a polypeptide with, or to, SEQ ID NO:2 is determined by i) aligning the two amino acid sequences using the Needle program, with the BLOSUM62 substitution matrix, a gap opening penalty of 10, and a gap extension penalty of 0.5; ii) counting the number of exact matches in the alignment; iii) dividing the number of exact matches by the length of the shortest of the two amino acid sequences, and iv) converting the result of the division of iii) into percentage.

In the above hypothetical example, the number of exact matches is 6, the length of the shortest one of the two amino acid sequences is 12; accordingly the percentage of identity is 50%.

In particular aspects of the phytase of the invention, the degree of identity to SEQ ID NO:2, SEQ ID NO:4 and/or SEQ ID NO:6 is at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%. In still further particular embodiments, the degree of identity is at least 98.0%, 98.2%, 98.4%, 98.6%, 98.8%, 99.0%, 99.1%, 99.2%, 99.3%, 99.4%, 99.5%, 99.6%, 99.7%, 99.8%, or at least 99.9%. In alternative embodiments, the degree of identity is at least 70%, 71%, 72%, or at least 73%.

In still further particular aspects the phytase of the invention has no more than 2, 3, 4, 5, 6, 7, 8, 9, or no more than 10 modifications as compared to SEQ ID NO:2 SEQ ID NO:4 and/or SEQ ID NO:6 or any other parent phytase; no more than 11, 12, 13, 14, 15, 16, 17, 18, 19, or no more than 20 modifications as compared to SEQ ID NO:2; no more than 21, 22, 23, 24, 25, 26, 27, 28, 29, or no more than 30 modifications as compared to SEQ ID NO:2; no more than 31, 32, 33, 34, 35, 36, 37, 38, 39, or not more than 40 modifications as compared to SEQ ID NO:2, SEQ ID NO:4 and/or SEQ ID NO:6 or any other parent phytase; no more than 41, 42, 43, 44, 45, 46, 47, 48, 49, or no more than 50 modifications as compared to SEQ ID NO:2, SEQ ID NO:4 and/or SEQ ID NO:6 or any other parent phytase; no more than 51, 52, 53, 54, 55, 56, 57, 58, 59, or no more than 60 modifications as compared to SEQ ID NO:2, SEQ ID NO:4 and/or SEQ ID NO:6 or any other parent phytase; no more than 61, 62, 63, 64, 65, 66, 67, 68, 69, or no more than 70 modifications as compared to SEQ ID NO:2, SEQ ID NO:4 and/or SEQ ID NO:6 or any other parent phytase; no more than 71, 72, 73, 74, 75, 76, 77, 78, 79, or no more than 80 modifications as compared to SEQ ID NO:2, SEQ ID NO:4 and/or SEQ ID NO:6 or any other parent phytase; no more than 81, 82, 83, 84, 85, 86, 87, 88, 89, or no more than 90 modifications as compared to SEQ ID NO:2, SEQ ID NO:4 and/or SEQ ID NO:6 or any other parent phytase; no more than 91, 92, 93, 94, 95, 96, 97, 98, 99, or no more than 100 modifications as compared to SEQ ID NO:2, SEQ ID NO:4 and/or SEQ ID NO:6 or any other parent phytase; no more than 101, 102, 103, 104, 105, 106, 107, 108, 109, or no more than 110 modifications as compared to SEQ ID NO:2, SEQ ID NO:4 and/or SEQ ID NO:6 or any other parent phytase; no more than 111, 112, 113, 114, 115, 116, 117, 118, 119, or no more than 120 modifications as compared to SEQ ID NO:2, SEQ ID NO:4 and/or SEQ ID NO:6 or any other parent phytase; or no more than 121, 122, 123, or 124 modifications as compared to SEQ ID NO:2, SEQ ID NO:4 and/or SEQ ID NO:6 or any other parent phytase.

### Position Numbering

The nomenclature used herein for defining amino acid positions is based on the amino acid sequence of the mature phytase of E. coli deposited in UNIPROT under the id. A7ZK83, which is given in the sequence lisiting as SEQ ID NO:2 (amino acids 1-412 of SEQ ID NO:2). Accordingly, in the present context, the basis for numbering positions is SEQ ID NO:2 starting with Q1 and ending with S412. (SEQ ID NO:2) as the standard for position numbering and, thereby, also for the nomenclature.

When used herein the term "mature" part (or sequence) refers to that part of the polypeptide which is secreted by a cell which contains, as part of its genetic equipment, a polynucleotide encoding the polypeptide. In other words, the mature polypeptide part refers to that part of the polypeptide which remains after the signal peptide part, as well as a propeptide part, if any, has been cleaved off. The signal peptide part can be predicted by programs known in the art (e.g. SignaIP). Generally, the first amino acid of the mature part of an enzyme can be determined by N-terminal sequencing of the purified enzyme. Any difference between the signal peptide part and the mature part must then be due to to the presence of a propeptide.

### Modifications, such as Substitutions, Deletions, Insertions

A phytase variant can comprise various types of modifications relative to a template (i.e. a reference or comparative amino acid sequence such as SEQ ID NO:2): An amino acid can be substituted with another amino acid; an amino acid can be deleted; an amino acid can be inserted; as well as any combination of any number of such modifications. In the present context the term "insertion" is intended to cover also N- and/or C-terminal extensions.

The general nomenclature used herein for a single modification is the following: XDcY, where "X" and "Y" independently designate a one-letter amino acid code, or a "*" (deletion of an amino acid), "D" designates a number, and "c" designates an alphabetical counter (a, b, c, and so forth), which is only present in insertions. Reference is made to Table 1 below which describes purely hypothetical examples of applying this nomenclature to various types of modifications.

**Table 1**

| Type | Description | Example |
|---|---|---|
| Substitution | X=Amino acid in template | **G80A** |
| | D=Position in template c empty | |
| | Y=Amino acid in variant | |
| Insertion | X="*" | ***80aT *80bY *85aS** |
| | D=Position in template before the insertion c="a" for first insertion at this position, "b" for next, etc | |
| Deletion | X=Amino acid in template | **V81*** |
| | D=Position in template c empty | |
| | Y="*" | |
| N-terminal extension | Insertions at position "0". | ***0aA *0bT *0cG** |
| | | |
| C-terminal extension | Insertions after the N-terminal amino acid. | ***275aS *275bT** |
| | | |

As explained above, the position number ("D") is counted from the first amino acid residue of SEQ ID NO:2.

Several modifications in the same sequence are separated by "/" (slash), e.g. the designation "1*/2*/3*" means that the amino acids in position number 1, 2, and 3 are all deleted, and the designation "104A/105F" means that the amino acid in position number 104 is substituted by A, and the amino acid in position number 105 is substituted by F.

Alternative modifications are separated by "," (comma), e.g., the designation "119R,K" means that the amino acid in position 119 is substituted with R or K.

The commas used herein in various other enumerations of possibilities mean what they usually do grammatically, viz. often and/or. E.g., the first comma in the listing "53V,Q, 121D, and/or 167Q" denotes an alternative (V or Q), whereas the two next commas should be interpreted as and/or options: 53 V or Q, and/or 121D, and/or 167Q.

In the present context, "at least one" (e.g. modification) means one or more, e.g. 1, 2, 3, 4, 5, 6, 7, 8, 9, or 10 modifications; or 12, 14, 15, 16, 18, 20, 22, 24, 25, 28, or 30 modifications; and so on, up to a maximum number of modifications of 125, 130, 140, 150, 160, 170, 180, 190, or of 200. The phytase variants of the invention, however, still have to be at least 85% identical to SEQ ID NO:2, this percentage being determined as described above.

A substitution or extension without any indication of what to substitute or extend with refers to the insertion of any natural, or non-natural, amino acid, except the one that occupies this position in the template.

### Identifying Corresponding Position Numbers

As explained above, the mature the amino acid sequence of the mature E. coli phytase deposited in UNIPROT under the id. A7ZK83 (SEQ ID NO:2) is used as the standard for position numbering and, thereby, also for the nomenclature.

For another phytase, in particular a phytase variant of the invention, the position corresponding to position D in SEQ ID NO:2 is found by aligning the two sequences as specified above in the section entitled "Phytase polypeptides, percentage of identity". From the alignment, the position in the sequence of the invention corresponding to position D of SEQ ID NO:2 can be clearly and unambiguously identified (the two positions on top of each other in the alignment).

Below some additional, purely hypothetical, examples are included which are derived from Table 1 above which in the third column includes a number of alignments of two sequences:
Consider the third cell in the first row of Table 1: The upper sequence is the template, the lower the variant. Position number 80 refers to amino acid residue G in the template. Amino acid A occupies the corresponding position in the variant. Accordingly, this substitution is designated G80A.

Consider now the third cell in the second row of Table 1: The upper sequence is again the template and the lower the variant. Position number 80 again refers to amino acid residue G in the template. The variant has two insertions, viz. TY, after G80 and before V81 in the template. Whereas the T and Y of course would have their own "real" position number in the variant amino acid sequence, for the present purposes we always refer to the template position numbers, and accordingly the T and the Y are said to be in position number 80a and 80b, respectively.

Finally, consider the third cell in the last row of Table 1: Position number 275 refers to the last amino acid of the template. A C-terminal extension of ST are said to be in position number 275a and 275b, respectively, although, again, of course they have their own "real" position number in the variant amino acid sequence.

### Modified Properties, Reference Phytase

In a particular aspect, the phytase of the disclosure has modified, preferably improved, properties. The terms "modified" and "improved" imply a comparison with another phytase. Examples of such other, reference, or comparative, phytases are: SEQ ID NO:3, SEQ ID NO:4, SEQ ID NO:5, and/or SEQ ID NO:6.

Non-limiting examples of properties that are modified, preferably improved, are the following: Thermostability, pH profile, specific activity, performance in animal feed, protease-sensibility, and/or glycosylation pattern. The phytase of the disclosure may also have an modified, preferably improved, temperature profile, and/or it may incorporate a change of a potential protease cleavage site.

### Thermal Performance,

### Thermostability

Thermostability may be determined as described in Example 3, i.e. using DSC measurements to determine the denaturation temperature, Td, of the purified phytase protein. The Td is indicative of the thermostability of the protein: The higher the Td, the higher the thermostability. Accordingly, in a preferred embodiment, the phytase of the invention has a Td which is higher than the Td of a reference phytase, wherein Td is determined on purified phytase samples (preferably with a purity of at least 90% or 95%, determined by SDS-PAGE).

Thermostability may also be determined as follows. Accordingly, in a preferred embodiment the phytase of the invention, after incubation for 60 minutes at 70°C and pH 4.0, has an improved residual activity as compared to the residual activity of a reference phytase treated in the same way, the residual activity being calculated for each phytase relative to the activity found before the incubation (at 0 minutes). The residual activity is preferably measured on sodium phytate at pH 5.5 and 37°C. The incubation is preferably in 0.1 M sodium acetate, pH 4.0. The phytase is preferably purified, more preferably to a purity of at least 95%, determined by SDS-PAGE. A preferred phytase activity assay buffer is 0.25 M Na-acetate pH 5.5. Using this method, the residual activity of the phytase of the invention is preferably at least 105% of the residual activity of the reference phytase, more preferably at least 110%, 115%, 120%, 130%, 140%, 150%, 160%, 170%, 180%, 190%, or at least 200%. In the alternative, the residual activity relative to the activity at 0 minutes is preferably at least 31%, or at least 32%. The following substitutions providing improved thermostability stability are preferred (see Table 9): 273L, 46E, 362R, and/or 53V.

In a particular embodiment, the phytase variant of the invention is more thermostable than the reference phytase, wherein thermostability is determined using any of the above-mentioned four tests (based on the Examples).

### Heat-stability,

Heat stability may be determined as described in Example 4 by determining the temperature/activity profile of the variant phytases.

### Temperature profile/temperature stability,

Whether or not a phytase of the invention has a modified temperature profile as compared to a reference phytase may be determined as described in Example 4. Accordingly, in a particular embodiment the phytase of the invention has a modified temperature profile as compared to a reference phytase, wherein the temperature profile is determined as phytase activity as a function of temperature on sodium phytate at pH 5.5 in the temperature range of 20-90°C (in 10°C steps). A preferred buffer is in 0.25 M Na-acetate buffer pH 5.5. The activity at each temperature is preferably indicated as relative activity (in %) normalized to the value at optimum temperature. The optimum temperature is that temperature within the tested temperatures (i.e. those with 5-10°C jumps) where the activity is highest.

### pH profile

Whether or not a phytase of the disclosure has an modified pH profile as compared to a reference phytase may be determined as described in the Examples. Accordingly, in a particular embodiment the phytase of the invention has an modified pH profile as compared to a reference phytase, wherein the pH profile is determined as phytase activity as a function of pH on sodium phytate at 37°C in the pH range of 2.0 to 7.5 (in 0.5 pH-unit steps). A preferred buffer is a cocktail of 50mM glycine, 50mM acetic acid and 50mM Bis-Tris. Another preferred buffer is 0.25M sodium acetate. The activity at each pH is preferably indicated as relative activity (in %) normalized to the value at optimum pH.

An example of an modified pH profile is where the pH curve (relative activity as a function of pH) is shifted towards higher, or lower, pH.

Another example of an modified pH profile is where the optimum pH is changed, in the upward or the downward direction

A modified pH profile may also be determined by comparing phosphatase activity at pH 3.5 and 5.5. Alternatively, the activity at pH 3.5 may be compared with the activity at pH 4.0, 4.5, or 5.0. In a still further alternative embodiment, phytase activities are compared instead of phosphatase activities.

In a particular embodiment, the phytase of the disclosure has an modified pH profile as compared to a reference phytase. More in particular, the pH profile is modified in the pH-range of 3.5-5.5. Still more in particular, the activity at pH 4.0, 4.5, 5.0, and/or 5.5 is at a level of at least 50%, 55%, 60%, 65%, 70%, 75%, 80%, 85%, 90%, or at least 95% of the activity at the pH-optimum.

### Specific Activity

In a particular disclosure, the phytase of the invention has an improved specific activity relative to a reference phytase. More in particular, the specific activity of a phytase of the disclosure is at least 105%, relative to the specific activity of a reference phytase determined by the same procedure. In still further particular aspects, the relative specific activity is at least 110, 115, 120, 125, 130, 140, 145, 150, 160, 170, 180, 190, 200, 220, 240, 260, 280, 300, 350 or even 400%, still relative to the specific activity of the reference phytase as determined by the same procedure.

In the alternative, the term high specific activity refers to a specific activity of at least 200 FYT/mg Enzyme Protein (EP). In particular aspects, the specific activity is at least 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900 or 3000 FYT/mg EP.

Specific activity is measured on highly purified samples (an SDS poly acryl amide gel should show the presence of only one component). The enzyme protein concentration may be determined by amino acid analysis, and the phytase activity in the units of FYT, determined as described in the Examples. Specific activity is a characteristic of the specific phytase variant in question, and it is calculated as the phytase activity measured in FYT units per mg phytase variant enzyme protein.

### Performance in animal feed

In a particular aspect the phytase of the disclosure has an improved performance in animal feed as compared to a reference phytase. The performance in animal feed may be determined by an in vitro model of Example 6. Accordingly, in a preferred aspect the phytase of the invention has an improved performance in animal feed, wherein the performance is determined in an in vitro model, by preparing feed samples composed of 30% soybean meal and 70% maize meal with added CaCl₂ to a concentration of 5 g calcium per kg feed; pre-incubating them at 40°C and pH 3.0 for 30 minutes followed by addition of pepsin (3000 U/g feed) and phytase; incubating the samples at 40°C and pH 3.0 for 60 minutes followed by pH 4.0 for 30 minutes; stopping the reactions; extracting phytic acid and inositol-phosphates by addition of HCI to a final concentration of 0.5M and incubation at 40°C for 2 hours, followed by one freeze-thaw cycle and 1 hour incubation at 40°C; separating phytic acid and inositol-phosphates by high performance ion chromatography; determining the amount of residual phytate phosphorus (IP6-P); calculating the difference in residual IP6-P between the phytase-treated and a non-phytase-treated blank sample (this difference is degraded IP6-P); and expressing the degraded IP6-P of the phytase of the disclosure relative to degraded IP6-P of the reference phytase (e.g. the phytases having SEQ ID NO:3 and 4).

The phytase of the invention and the reference phytase are of course dosed in the same amount, preferably based on phytase activity units (FYT). A preferred dosage is 125 FYT/kg feed. Another preferred dosage is 250 FYT/kg feed. The phytases may be dosed in the form of purified phytases, or in the form of fermentation supernatants. Purified phytases preferably have a purity of at least 95%, as determined by SDS-PAGE.

In preferred aspects, the degraded IP6-P value of the purified phytase of the invention, relative to the degraded IP6-P value of the reference phytase, is at least 101%, or at least 102%, 103%, 104%, 105%, 110%, 115%, or at least 120%. In still further preferred embodiments, the degraded IP6-P value of the purified phytase of the invention, relative to the degraded IP6-P value of the reference phytase, is at least 125%, 130%, 140%, 150%, 160%, 170%, 180%, 190%, or at least 200%. Preferably, the degraded IP6-P value of the phytase of the invention, relative to the degraded IP6-P value of the SEQ ID NO:2 phytase, is at least 105%, 110%, 113%, 115%, 120%, 125%, or at least 130%.

The relative performance of a phytase of the invention may also be calculated as the percentage of the phosphorous released by the reference phytase.

In a still further particular aspect, the relative performance of the phytase of the invention may be calculated as the percentage of the phosphorous released by the phytase of the invention, relative to the amount of phosphorous released by the reference phytase.

In still further particular aspects, the relative performance of the phytase of the invention is at least 105%, preferably at least 110, 120, 130, 140, 150, 160, 170, 180, 190, or at least 200%.

### Nucleic Acid Sequences and Constructs

The present invention also relates to nucleic acid sequences comprising a nucleic acid sequence which encodes a phytase variant of the invention.

The term "isolated nucleic acid sequence" refers to a nucleic acid sequence which is essentially free of other nucleic acid sequences, e.g., at least about 20% pure, preferably at least about 40% pure, more preferably at least about 60% pure, even more preferably at least about 80% pure, and most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

The nucleic acid sequences of the invention can be prepared by introducing at least one mutation into a template phytase coding sequence or a subsequence thereof, wherein the mutant nucleic acid sequence encodes a variant phytase. The introduction of a mutation into the nucleic acid sequence to exchange one nucleotide for another nucleotide may be accomplished by any of the methods known in the art, e.g. by site-directed mutagenesis, by random mutagenesis, or by doped, spiked, or localized random mutagenesis.

Random mutagenesis is suitably performed either as localized or region-specific random mutagenesis in at least three parts of the gene translating to the amino acid sequence shown in question, or within the whole gene. When the mutagenesis is performed by the use of an oligonucleotide, the oligonucleotide may be doped or spiked with the three non-parent nucleotides during the synthesis of the oligonucleotide at the positions which are to be changed. The doping or spiking may be performed so that codons for unwanted amino acids are avoided. The doped or spiked oligonucleotide can be incorporated into the DNA encoding the phytase enzyme by any technique, using, e.g., PCR, LCR or any DNA polymerase and ligase as deemed appropriate.

Preferably, the doping is carried out using "constant random doping", in which the percentage of wild-type and mutation in each position is predefined. Furthermore, the doping may be directed toward a preference for the introduction of certain nucleotides, and thereby a preference for the introduction of one or more specific amino acid residues. The doping may be made, e.g., so as to allow for the introduction of 90% wild type and 10% mutations in each position. An additional consideration in the choice of a doping scheme is based on genetic as well as protein-structural constraints.

The random mutagenesis may be advantageously localized to a part of the parent phytase in question. This may, e.g., be advantageous when certain regions of the enzyme have been identified to be of particular importance for a given property of the enzyme.

Alternative methods for providing variants of the disclosure include gene shuffling e.g. as described in WO 95/22625 or in WO 96/00343, and the consensus derivation process as described in EP 897985.

### Nucleic Acid Constructs

A nucleic acid construct comprises a nucleic acid sequence of the present invention operably linked to one or more control sequences which direct the expression of the coding sequence in a suitable host cell under conditions compatible with the control sequences. Expression will be understood to include any step involved in the production of the polypeptide including, but not limited to, transcription, post-transcriptional modification, translation, post-translational modification, and secretion.

The term "nucleic acid construct" as used herein refers to a nucleic acid molecule, either single- or double-stranded, which is isolated from a naturally occurring gene or which is modified to contain segments of nucleic acids in a manner that would not otherwise exist in nature. The term nucleic acid construct is synonymous with the term "expression cassette" when the nucleic acid construct contains the control sequences required for expression of a coding sequence of the present invention.

The term "control sequences" is defined herein to include all components, which are necessary or advantageous for the expression of a polynucleotide encoding a polypeptide of the present invention. Each control sequence may be native or foreign to the nucleotide sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a polypeptide.

The term "operably linked" denotes herein a configuration in which a control sequence is placed at an appropriate position relative to the coding sequence of the polynucleotide sequence such that the control sequence directs the expression of the coding sequence of a polypeptide.

When used herein the term "coding sequence" (CDS) means a nucleotide sequence, which directly specifies the amino acid sequence of its protein product. The boundaries of the coding sequence are generally determined by an open reading frame, which usually begins with the ATG start codon or alternative start codons such as GTG and TTG. The coding sequence may a DNA, cDNA, or recombinant nucleotide sequence

### Expression Vector

The term "expression" includes any step involved in the production of the polypeptide including, but not limited to, transcription, post-transcriptional modification, translation, post-translational modification, and secretion.

The term "expression vector" is defined herein as a linear or circular DNA molecule that comprises a polynucleotide encoding a polypeptide of the invention, and which is operably linked to additional nucleotides that provide for its expression.

A nucleic acid sequence encoding a phytase variant of the invention can be expressed using an expression vector which typically includes control sequences encoding a promoter, operator, ribosome binding site, translation initiation signal, and, optionally, a repressor gene or various activator genes.

The recombinant expression vector carrying the DNA sequence encoding a phytase variant of the invention may be any vector which may conveniently be subjected to recombinant DNA procedures, and the choice of vector will often depend on the host cell into which it is to be introduced. The vector may be one which, when introduced into a host cell, is integrated into the host cell genome and replicated together with the chromosome(s) into which it has been integrated.

The phytase variant may also be co-expressed together with at least one other enzyme of animal feed interest, such as a phytase, phosphatase, xylanase, galactanase, alpha-galactosidase, protease, phospholipase, amylase, and/or beta-glucanase. The enzymes may be co-expressed from different vectors, from one vector, or using a mixture of both techniques. When using different vectors, the vectors may have different selectable markers, and different origins of replication. When using only one vector, the genes can be expressed from one or more promoters. If cloned under the regulation of one promoter (di- or multi-cistronic), the order in which the genes are cloned may affect the expression levels of the proteins. The phytase variant may also be expressed as a fusion protein, i.e. that the gene encoding the phytase variant has been fused in frame to the gene encoding another protein. This protein may be another enzyme or a functional domain from another enzyme.

### Host Cells

The term "host cell", as used herein, includes any cell type which is susceptible to transformation, transfection, transduction, and the like with a nucleic acid construct comprising a polynucleotide of the present invention.

The present invention also relates to recombinant host cells, comprising a polynucleotide of the present invention, which are advantageously used in the recombinant production of the polypeptides. A vector comprising a polynucleotide of the present invention is introduced into a host cell so that the vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be a unicellular microorganism, e.g., a prokaryote, or a non-unicellular microorganism, e.g., a eukaryote.

Useful unicellular microorganisms are bacterial cells such as gram positive bacteria including, but not limited to, a Bacillus cell, e.g., Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis, and Bacillus thuringiensis; or a Streptomyces cell, e.g., Streptomyces lividans and Streptomyces murinus, or gram negative bacteria such as E. coli and Pseudomonas sp. In a preferred aspect, the bacterial host cell is a Bacillus lentus, Bacillus licheniformis, Bacillus stearothermophilus, or Bacillus subtilis cell. In another preferred aspect, the Bacillus cell is an alkalophilic Bacillus.

The introduction of a vector into a bacterial host cell may, for instance, be effected by protoplast transformation (see, e.g., Chang and Cohen, 1979, Molecular General Genetics 168: 111-115), using competent cells (see, e.g., Young and Spizizin, 1961, Journal of Bacteriology 81: 823-829, or Dubnau and Davidoff-Abelson, 1971, Journal of Molecular Biology 56: 209-221), electroporation (see, e.g., Shigekawa and Dower, 1988, Biotechniques 6: 742-751), or conjugation (see, e.g., Koehler and Thorne, 1987, Journal of Bacteriology 169: 5771-5278).

The host cell may also be a eukaryote, such as a mammalian, insect, plant, or fungal cell.

In a preferred aspect, the host cell is a fungal cell. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth et al., 1995, supra, page 171) and all mitosporic fungi (Hawksworth et al., 1995, supra).

In a more preferred aspect, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, F.A., Passmore, S.M., and Davenport, R.R., eds, Soc. App. Bacteriol. Symposium Series No. 9, 1980).

In an even more preferred aspect, the yeast host cell is a Candida, Hansenula, Kluyveromyces, Pichia, Saccharomyces, Schizosaccharomyces, or Yarrowia cell.

In a most preferred aspect, the yeast host cell is a Pichia pastoris, Pichia methanolica, Saccharomyces carlsbergensis, Saccharomyces cerevisiae, Saccharomyces diastaticus, Saccharomyces douglasii, Saccharomyces kluyveri, Saccharomyces norbensis or Saccharomyces oviformis cell. In another most preferred aspect, the yeast host cell is a Kluyveromyces lactis cell. In another most preferred aspect, the yeast host cell is a Yarrowia lipolytica cell.

In another more preferred aspect, the fungal host cell is a filamentous fungal cell. "Filamentous fungi" include all filamentous forms of the subdivision Eumycota and Oomycota (as defined by Hawksworth et al., 1995, supra). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as Saccharomyces cerevisiae is by budding of a unicellular thallus and carbon catabolism may be fermentative.

In an even more preferred aspect, the filamentous fungal host cell is an Acremonium, Aspergillus, Aureobasidium, Bjerkandera, Ceriporiopsis, Coprinus, Coriolus, Cryptococcus, Filobasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, or Trichoderma cell.

In a most preferred aspect, the filamentous fungal host cell is an Aspergillus awamori, Aspergillus fumigatus, Aspergillus foetidus, Aspergillus japonicus, Aspergillus nidulans, Aspergillus niger or Aspergillus oryzae cell. In another most preferred aspect, the filamentous fungal host cell is a Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, or Fusarium venenatum cell. In another most preferred aspect, the filamentous fungal host cell is a Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, or Ceriporiopsis subvermispora, Coprinus cinereus, Coriolus hirsutus, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei, or Trichoderma viride strain cell.

Fungal cells may be transformed by a process involving protoplast formation, transformation of the protoplasts, and regeneration of the cell wall in a manner known per se. Suitable procedures for transformation of Aspergillus and Trichoderma host cells are described in EP 238 023 and Yelton et al., 1984, Proceedings of the National Academy of Sciences USA 81: 1470-1474. Suitable methods for transforming Fusarium species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

### Methods of Production

The present invention also relates to methods for producing a phytase of the present invention comprising (a) cultivating a host cell under conditions conducive for production of the phytase; and (b) recovering the phytase.

In the production methods of the present invention, the cells are cultivated in a nutrient medium suitable for production of the polypeptide using methods well known in the art. For example, the cell may be cultivated by shake flask cultivation, and small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.

The resulting polypeptide may be recovered using methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989).

### Transgenic Plants

The present disclosure also relates to a transgenic plant, plant part, or plant cell which has been transformed with a nucleotide sequence encoding a polypeptide having phytase activity of the present invention so as to express and produce the polypeptide in recoverable quantities. The polypeptide may be recovered from the plant or plant part. Alternatively, the plant or plant part containing the recombinant polypeptide may be used as such for improving the quality of a food or feed, e.g., improving nutritional value, palatability, and rheological properties, or to destroy an antinutritive factor.

In a particular aspect, the polypeptide is targeted to the endosperm storage vacuoles in seeds. This can be obtained by synthesizing it as a precursor with a suitable signal peptide, see Horvath et al in PNAS, Feb. 15, 2000, vol. 97, no. 4, p. 1914-1919.

The transgenic plant can be dicotyledonous (a dicot) or monocotyledonous (a monocot) or engineered variants thereof. Examples of monocot plants are grasses, such as meadow grass (blue grass, Poa), forage grass such as Festuca, Lolium, temperate grass, such as Agrostis, and cereals, e.g., wheat, oats, rye, barley, rice, sorghum, triticale (stabilized hybrid of wheat (Triticum) and rye (Secale), and maize (corn). Examples of dicot plants are tobacco, legumes, such as sunflower (Helianthus), cotton (Gossypium), lupins, potato, sugar beet, pea, bean and soybean, and cruciferous plants (family Brassicaceae), such as cauliflower, rape seed, and the closely related model organism Arabidopsis thaliana. Low-phytate plants as described e.g. in US patent no. 5,689,054 and US patent no. 6,111,168 are examples of engineered plants.

Examples of plant parts are stem, callus, leaves, root, fruits, seeds, and tubers, as well as the individual tissues comprising these parts, e.g. epidermis, mesophyll, parenchyma, vascular tissues, meristems. Also specific plant cell compartments, such as chloroplast, apoplast, mitochondria, vacuole, peroxisomes, and cytoplasm are considered to be a plant part. Furthermore, any plant cell, whatever the tissue origin, is considered to be a plant part. Likewise, plant parts such as specific tissues and cells isolated to facilitate the utilisation of the disclosure are also considered plant parts, e.g. embryos, endosperms, aleurone and seed coats. Also included within the scope of the present disclosure are the progeny of such plants, plant parts and plant cells.

The transgenic plant or plant cell expressing a polypeptide of the present invention may be constructed in accordance with methods known in the art. Briefly, the plant or plant cell is constructed by incorporating one or more expression constructs encoding a polypeptide of the present invention into the plant host genome and propagating the resulting modified plant or plant cell into a transgenic plant or plant cell.

Conveniently, the expression construct is a nucleic acid construct which comprises a nucleic acid sequence encoding a polypeptide of the present invention operably linked with appropriate regulatory sequences required for expression of the nucleic acid sequence in the plant or plant part of choice. Furthermore, the expression construct may comprise a selectable marker useful for identifying host cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences are determined, for example, on the basis of when, where, and how the polypeptide is desired to be expressed. For instance, the expression of the gene encoding a polypeptide of the present invention may be constitutive or inducible, or may be developmental, stage or tissue specific, and the gene product may be targeted to a specific cell compartment, tissue or plant part such as seeds or leaves. Regulatory sequences are, for example, described by Tague et al., 1988, Plant Physiology 86: 506.

For constitutive expression, the following promoters may be used: The 35S-CaMV promoter (Franck et al., 1980, Cell 21: 285-294), the maize ubiquitin 1 (Christensen AH, Sharrock RA and Quail 1992. Maize polyubiquitin genes: structure, thermal perturbation of expression and transcript splicing, and promoter activity following transfer to protoplasts by electroporation), or the rice actin 1 promoter (Plant Mo. Biol. 18, 675-689.; Zhang W, McElroy D. and Wu R 1991, Analysis of rice Act1 5' region activity in transgenic rice plants. Plant Cell 3, 1155-1165). Organ-specific promoters may be, for example, a promoter from storage sink tissues such as seeds, potato tubers, and fruits (Edwards & Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant and Cell Physiology 39: 885-889), a Vicia faba promoter from the legumin B4 and the unknown seed protein gene from Vicia faba (Conrad et al., 1998, Journal of Plant Physiology 152: 708-711), a promoter from a seed oil body protein (Chen et al., 1998, Plant and Cell Physiology 39: 935-941), the storage protein napA promoter from Brassica napus, or any other seed specific promoter known in the art, e.g., as described in WO 91/14772. Furthermore, the promoter may be a leaf specific promoter such as the rbcs promoter from rice or tomato (Kyozuka et al., 1993, Plant Physiology 102: 991-1000, the chlorella virus adenine methyltransferase gene promoter (Mitra and Higgins, 1994, Plant Molecular Biology 26: 85-93), or the aldP gene promoter from rice (Kagaya et al., 1995, Molecular and General Genetics 248: 668-674), or a wound inducible promoter such as the potato pin2 promoter (Xu et al., 1993, Plant Molecular Biology 22: 573-588). Likewise, the promoter may be inducible by abiotic treatments such as temperature, drought or modifications in salinity or inducible by exogenously applied substances that activate the promoter, e.g. ethanol, oestrogens, plant hormones like ethylene, abscisic acid, gibberellic acid, and/or heavy metals.

A promoter enhancer element may also be used to achieve higher expression of the polypeptide in the plant. For instance, the promoter enhancer element may be an intron which is placed between the promoter and the nucleotide sequence encoding a polypeptide of the present invention. For instance, Xu et al., 1993, supra disclose the use of the first intron of the rice actin 1 gene to enhance expression.

Still further, the codon usage may be optimized for the plant species in question to improve expression (see Horvath et al referred to above).

The selectable marker gene and any other parts of the expression construct may be chosen from those available in the art.

The nucleic acid construct is incorporated into the plant genome according to conventional techniques known in the art, including Agrobacterium-mediated transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

Presently, Agrobacterium tumefaciens-mediated gene transfer is the method of choice for generating transgenic dicots (for a review, see Hooykas and Schilperoort, 1992, Plant Molecular Biology 19: 15-38), and it can also be used for transforming monocots, although other transformation methods are more often used for these plants. Presently, the method of choice for generating transgenic monocots, supplementing the Agrobacterium approach, is particle bombardment (microscopic gold or tungsten particles coated with the transforming DNA) of embryonic calli or developing embryos (Christou, 1992, Plant Journal 2: 275-281; Shimamoto, 1994, Current Opinion Biotechnology 5: 158-162; Vasil et al., 1992, Bio/Technology 10: 667-674). An alternative method for transformation of monocots is based on protoplast transformation as described by Omirulleh et al., 1993, Plant Molecular Biology 21: 415-428.

Following transformation, the transformants having incorporated therein the expression construct are selected and regenerated into whole plants according to methods well-known in the art. Often the transformation procedure is designed for the selective elimination of selection genes either during regeneration or in the following generations by using e.g. co-transformation with two separate T-DNA constructs or site specific excision of the selection gene by a specific recombinase.

The present disclosure also relates to methods for producing a polypeptide of the present invention comprising (a) cultivating a transgenic plant or a plant cell comprising a nucleic acid sequence encoding a polypeptide having phytase activity of the present invention under conditions conducive for production of the polypeptide; and (b) recovering the polypeptide.

### Compositions and Uses

In still further aspects, the present invention relates to compositions comprising a polypeptide of the present invention, as well as methods of using these.

The polypeptide compositions may be prepared in accordance with methods known in the art and may be in the form of a liquid or a dry composition. For instance, the polypeptide composition may be in the form of granulates or microgranulates. The polypeptide to be included in the composition may be stabilized in accordance with methods known in the art.

The phytase of the invention can be used for degradation, in any industrial context, of, for example, phytate, phytic acid, and/or the mono-, di-, tri-, tetra- and/or penta-phosphates of myo-inositol. It is well known that the phosphate moieties of these compounds chelates divalent and trivalent cations such as metal ions, i.a. the nutritionally essential ions of calcium, iron, zinc and magnesium as well as the trace minerals manganese, copper and molybdenum. Besides, the phytic acid also to a certain extent binds proteins by electrostatic interaction.

Accordingly, preferred uses of the polypeptides of the invention are in animal feed preparations (including human food) or in additives for such preparations.

In a particular aspect, the polypeptide of the invention can be used for improving the nutritional value of an animal feed. Non-limiting examples of improving the nutritional value of animal feed (including human food), are: Improving feed digestibility; promoting growth of the animal; improving feed utilization; improving bio-availability of proteins; increasing the level of digestible phosphate; improving the release and/or degradation of phytate; improving bio-availability of trace minerals; improving bio-availability of macro minerals; eliminating the need for adding supplemental phosphate, trace minerals, and/or macro minerals; and/or improving egg shell quality. The nutritional value of the feed is therefore increased, and the growth rate and/or weight gain and/or feed conversion (i.e. the weight of ingested feed relative to weight gain) of the animal may be improved.

Furthermore, the polypeptide of the invention can be used for reducing phytate level of manure.

### Animals, Animal Feed, and Animal Feed Additives

The term animal includes all animals, including human beings. Examples of animals are non-ruminants, and ruminants. Ruminant animals include, for example, animals such as sheep, goat, and cattle, e.g. cow such as beef cattle and dairy cows. In a particular aspect, the animal is a non-ruminant animal. Non-ruminant animals include mono-gastric animals, e.g. pig or swine (including, but not limited to, piglets, growing pigs, and sows); poultry such as turkeys, ducks and chickens (including but not limited to broiler chicks, layers); fish (including but not limited to salmon, trout, tilapia, catfish and carp); and crustaceans (including but not limited to shrimp and prawn).

The term feed or feed composition means any compound, preparation, mixture, or composition suitable for, or intended for intake by an animal.

In the use according to the the polypeptide can be fed to the animal before, after, or simultaneously with the diet. The latter is preferred.

In a particular aspect, the polypeptide, in the form in which it is added to the feed, or when being included in a feed additive, is substantially pure. In a particular aspect it is well-defined. The term "well-defined" means that the phytase preparation is at least 50% pure as determined by Size-exclusion chromatography (see Example 12 of WO 01/58275). In other particular aspects the phytase preparation is at least 60, 70, 80, 85, 88, 90, 92, 94, or at least 95% pure as determined by this method.

A substantially pure, and/or well-defined polypeptide preparation is advantageous. For instance, it is much easier to dose correctly to the feed a polypeptide that is essentially free from interfering or contaminating other polypeptides. The term dose correctly refers in particular to the objective of obtaining consistent and constant results, and the capability of optimising dosage based upon the desired effect.

For the use in animal feed, however, the phytase polypeptide of the invention need not be that pure; it may e.g. include other polypeptides, in which case it could be termed a phytase preparation.

The phytase preparation can be (a) added directly to the feed (or used directly in a treatment process of proteins), or (b) it can be used in the production of one or more intermediate compositions such as feed additives or premixes that is subsequently added to the feed (or used in a treatment process). The degree of purity described above refers to the purity of the original polypeptide preparation, whether used according to (a) or (b) above.

Polypeptide preparations with purities of this order of magnitude are in particular obtainable using recombinant methods of production, whereas they are not so easily obtained and also subject to a much higher batch-to-batch variation when the polypeptide is produced by traditional fermentation methods.

Such polypeptide preparation may of course be mixed with other polypeptides.

The polypeptide can be added to the feed in any form, be it as a relatively pure polypeptide, or in admixture with other components intended for addition to animal feed, i.e. in the form of animal feed additives, such as the so-called pre-mixes for animal feed.

In a further aspect the present invention relates to compositions for use in animal feed, such as animal feed, and animal feed additives, e.g. premixes.

Apart from the polypeptide of the invention, the animal feed additives of the disclosure contain at least one fat-soluble vitamin, and/or at least one water soluble vitamin, and/or at least one trace mineral. The feed additive may also contain at least one macro mineral.

Further, optional, feed-additive ingredients are colouring agents, e.g. carotenoids such as beta-carotene, astaxanthin, and lutein; aroma compounds; stabilisers; antimicrobial peptides; polyunsaturated fatty acids; reactive oxygen generating species; and/or at least one other polypeptide selected from amongst phytase (EC 3.1.3.8 or 3.1.3.26); phosphatase (EC 3.1.3.1; EC 3.1.3.2; EC 3.1.3.39); xylanase (EC 3.2.1.8); galactanase (EC 3.2.1.89); alpha-galactosidase (EC 3.2.1.22); protease (EC 3.4.-.-), phospholipase A1 (EC 3.1.1.32); phospholipase A2 (EC 3.1.1.4); lysophospholipase (EC 3.1.1.5); phospholipase C (3.1.4.3); phospholipase D (EC 3.1.4.4); amylase such as, for example, alpha-amylase (EC 3.2.1.1); and/or beta-glucanase (EC 3.2.1.4 or EC 3.2.1.6).

In a particular aspect these other polypeptides are well-defined (as defined above for phytase preparations).

The phytase of the invention may also be combined with other phytases, for example ascomycete phytases such as Aspergillus phytases, for example derived from Aspergillus ficuum, Aspergillus niger, or Aspergillus awamori; or basidiomycete phytases, for example derived from Peniophora lycii, Agrocybe pediades, Trametes pubescens, or Paxillus involutus; or derivatives, fragments or variants thereof which have phytase activity.

Thus, in preferred aspects of the use in animal feed of the invention, and in preferred aspects of the animal feed additive and the animal feed of the invention, the phytase of the invention is combined with such phytases.

Examples of antimicrobial peptides (AMP's) are CAP18, Leucocin A, Tritrpticin, Protegrin-1, Thanatin, Defensin, Lactoferrin, Lactoferricin, and Ovispirin such as Novispirin (Robert Lehrer, 2000), Plectasins, and Statins, including the compounds and polypeptides disclosed in WO 03/044049 and WO 03/048148, as well as variants or fragments of the above that retain antimicrobial activity.

Examples of antifungal polypeptides (AFP's) are the Aspergillus giganteus, and Aspergillus niger peptides, as well as variants and fragments thereof which retain antifungal activity, as disclosed in WO 94/01459 and WO 02/090384.

Examples of polyunsaturated fatty acids are C18, C20 and C22 polyunsaturated fatty acids, such as arachidonic acid, docosohexaenoic acid, eicosapentaenoic acid and gamma-linoleic acid.

Examples of reactive oxygen generating species are chemicals such as perborate, persulphate, or percarbonate; and polypeptides such as an oxidase, an oxygenase or a syntethase.

Usually fat- and water-soluble vitamins, as well as trace minerals form part of a so-called premix intended for addition to the feed, whereas macro minerals are usually separately added to the feed. Either of these composition types, when enriched with a polypeptide of the invention, is an animal feed additive of the disclosure.

In a particular aspect, the animal feed additive of the invention is intended for being included (or prescribed as having to be included) in animal diets or feed at levels of 0.01 to 10.0%; more particularly 0.05 to 5.0%; or 0.2 to 1.0% (% meaning g additive per 100 g feed). This is so in particular for premixes.

The following are non-exclusive lists of examples of these components:
Examples of fat-soluble vitamins are vitamin A, vitamin D3, vitamin E, and vitamin K, e.g. vitamin K3.

Examples of water-soluble vitamins are vitamin B12, biotin and choline, vitamin B1, vitamin B2, vitamin B6, niacin, folic acid and panthothenate, e.g. Ca-D-panthothenate.

Examples of trace minerals are manganese, zinc, iron, copper, iodine, selenium, and cobalt.

Examples of macro minerals are calcium, phosphorus and sodium.

The nutritional requirements of these components (exemplified with poultry and piglets/pigs) are listed in Table A of WO 01/58275. Nutritional requirement means that these components should be provided in the diet in the concentrations indicated.

In the alternative, the animal feed additive of the disclosure comprises at least one of the individual components specified in Table A of WO 01/58275. At least one means either of, one or more of, one, or two, or three, or four and so forth up to all thirteen, or up to all fifteen individual components. More specifically, this at least one individual component is included in the additive of the disclosure in such an amount as to provide an in-feed-concentration within the range indicated in column four, or column five, or column six of Table A.

The present invention also relates to animal feed compositions. Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore such fish diets usually have a crude fat content of 200-310 g/kg.

An animal feed composition according to the disclosure has a crude protein content of 50-800 g/kg, and furthermore comprises at least one polypeptide as claimed herein.

Furthermore, or in the alternative (to the crude protein content indicated above), the animal feed composition of the disclosure has a content of metabolisable energy of 10-30 MJ/kg; and/or a content of calcium of 0.1-200 g/kg; and/or a content of available phosphorus of 0.1-200 g/kg; and/or a content of methionine of 0.1-100 g/kg; and/or a content of methionine plus cysteine of 0.1-150 g/kg; and/or a content of lysine of 0.5-50 g/kg.

In particular aspects, the content of metabolisable energy, crude protein, calcium, phosphorus, methionine, methionine plus cysteine, and/or lysine is within any one of ranges 2, 3, 4 or 5 in Table B of WO 01/58275 (R. 2-5).

Crude protein is calculated as nitrogen (N) multiplied by a factor 6.25, i.e. Crude protein (g/kg)= N (g/kg) x 6.25. The nitrogen content is determined by the Kjeldahl method (A.O.A.C., 1984, Official Methods of Analysis 14th ed., Association of Official Analytical Chemists, Washington DC).

Metabolisable energy can be calculated on the basis of the NRC publication Nutrient requirements in swine, ninth revised edition 1988, subcommittee on swine nutrition, committee on animal nutrition, board of agriculture, national research council. National Academy Press, Washington, D.C., pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.

The dietary content of calcium, available phosphorus and amino acids in complete animal diets is calculated on the basis of feed tables such as Veevoedertabel 1997, gegevens over chemische samenstelling, verteerbaarheid en voederwaarde van voedermiddelen, Central Veevoederbureau, Runderweg 6, 8219 pk Lelystad. ISBN 90-72839-13-7.

In a particular aspect, the animal feed composition of the disclosure contains at least one protein. The protein may be an animal protein, such as meat and bone meal, and/or fish meal; or it may be a vegetable protein. The term vegetable proteins as used herein refers to any compound, composition, preparation or mixture that includes at least one protein derived from or originating from a vegetable, including modified proteins and protein-derivatives. In particular aspects, the protein content of the vegetable proteins is at least 10, 20, 30, 40, 50, or 60% (w/w).

Vegetable proteins may be derived from vegetable protein sources, such as legumes and cereals, for example materials from plants of the families Fabaceae (Leguminosae), Cruciferaceae, Chenopodiaceae, and Poaceae, such as soy bean meal, lupin meal and rapeseed meal.

In a particular aspect, the vegetable protein source is material from one or more plants of the family Fabaceae, e.g. soybean, lupine, pea, or bean.

In another particular aspect, the vegetable protein source is material from one or more plants of the family Chenopodiaceae, e.g. beet, sugar beet, spinach or quinoa.

Other examples of vegetable protein sources are rapeseed, sunflower seed, cotton seed, and cabbage.

Soybean is a preferred vegetable protein source.

Other examples of vegetable protein sources are cereals such as barley, wheat, rye, oat, maize (corn), rice, triticale, and sorghum.

In still further particular aspects, the animal feed composition of the disclosure contains 0-80% maize; and/or 0-80% sorghum; and/or 0-70% wheat; and/or 0-70% Barley; and/or 0-30% oats; and/or 0-40% soybean meal; and/or 0-25% fish meal; and/or 0-25% meat and bone meal; and/or 0-20% whey.

Animal diets can e.g. be manufactured as mash feed (non pelleted) or pelleted feed. Typically, the milled feed-stuffs are mixed and sufficient amounts of essential vitamins and minerals are added according to the specifications for the species in question. Polypeptides can be added as solid or liquid polypeptide formulations. For example, a solid polypeptide formulation is typically added before or during the mixing step; and a liquid polypeptide preparation is typically added after the pelleting step. The polypeptide may also be incorporated in a feed additive or premix.

The final polypeptide concentration in the diet is within the range of 0.01-200 mg polypeptide protein per kg diet, for example in the range of 5-30 mg polypeptide protein per kg animal diet.

The phytase of the invention should of course be applied in an effective amount, i.e. in an amount adequate for improving solubilisation and/or improving nutritional value of feed. It is at present contemplated that the polypeptide is administered in one or more of the following amounts (dosage ranges): 0.01-200; 0.01-100; 0.5-100; 1-50; 5-100; 10-100; 0.05-50; or 0.10-10 - all these ranges being in mg phytase polypeptide protein per kg feed (ppm).

For determining mg phytase polypeptide protein per kg feed, the phytase is purified from the feed composition, and the specific activity of the purified phytase is determined using a relevant assay. The phytase activity of the feed composition as such is also determined using the same assay, and on the basis of these two determinations, the dosage in mg phytase protein per kg feed is calculated.

The same principles apply for determining mg phytase polypeptide protein in feed additives. Of course, if a sample is available of the phytase used for preparing the feed additive or the feed, the specific activity is determined from this sample (no need to purify the phytase from the feed composition or the additive).

### Methods for Producing Fermentation Products

Yet another aspect of the present invention relates to the methods for producing a fermentation product, such as, e.g., ethanol, beer, wine, distillers dried grains (DDG), wherein the fermentation is carried out in the presence of a phytase produced by the present invention. Examples of fermentation processes include, for example, the processes described in WO 01/62947. Fermentation is carried out using a fermenting microorganism, such as, yeast.

In a particular aspect, the present disclosure provides methods for producing fermentation product, comprising (a) fermenting (using a fermenting microorganism, such as yeast) a carbohydrate containing material (e.g., starch) in the presence of a phytase of the present invention and (b) producing the fermentation product from the fermented carbohydrate containing material.

In a particular aspect, the present disclosure provides methods for producing ethanol, comprising fermenting (using a fermenting microorganism, such as yeast) a carbohydrate containing material (e.g., starch) in the presence of a phytase of the present invention and producing or recovering ethanol from the fermented carbohydrate containing material.

In another aspect, the present disclosure provides methods for producing ethanol comprising a) hydrolyzing starch, e.g., by a liquefaction and/or saccharification process, a raw starch hydrolysis process, b) fermenting the resulting starch in the presence of a phytase of the present invention, and c) producing ethanol.

The phytase may be added to the fermentation process at any suitable stage and in any suitable composition, including alone or in combination with other enzymes, such as, one or more alpha-amylases, glucoamylases, proteases, and/or cellulases.

In another aspect, the present disclosure provides methods for producing ethanol comprising hydrolyzing biomass, and fermenting (using a fermenting microorganism, such as yeast) the resulting biomass in the presence of a phytase of the present invention.

### Examples

Chemicals used are commercial products of at least reagent grade.

### Example 1: Preparation of variants, and determination of activity

### Preparation of phytase variants

### Expression of phytase variants in Aspergillus oryzae

The constructs comprising the E. coli phytase variant genes in the examples were used to construct expression vectors for *Aspergillus.* The Aspergillus expression vectors consist of an expression cassette based on the *Aspergillus niger* neutral amylase II promoter fused to the *Aspergillus nidulans* triose phosphate isomerase non translated leader sequence (Pna2/tpi) and the *Aspergillus niger* amyloglycosidase terminator (Tamg). Also present on the plasmid was the *Aspergillus* selective marker amdS from *Aspergillus nidulans* enabling growth on media for transformed aspergillus where acetamid is the sole nitrogen source. The expression plasmids for phytase variants were transformed into *Aspergillus* as described in Lassen et al. (2001), Applied and Environmental Microbiology, 67, 4701-4707. For each of the constructs 4-6 strains were isolated, purified and cultivated in microtiterplates. Expression was determined using a p-nitrophenyl phosphate substrate. The best producing strain was fermented in Shake flasks.

### Purification of E. coli phytase variants

The fermentation supernatant with the phytase variant was filtered through a sandwich of four Whatman glass microfibre filters (2.7, 1.6, 1.2 and 0.7 micrometer). Following this the solution was filtered through a Fast PES Bottle top filter with a 0.22 µm cut-off. The solution was added solid ammonium sulfate giving a final concentration of 1.5 M and the pH was adjusted to 6.0. The solution became a little cloudy and this precipitation was removed by filtration through a Fast PES Bottle top filter with a 0.22 µm cut-off.

The phytase-containing solution was applied to a butyl-sepharose column, approximately 50 ml in a XK26 column, using as buffer A 25 mM bis-tris (Bis-(2-hydroxyethyl)imino-tris(hydroxymethyl)methan)) + 1.5 M ammonium sulfate pH 6.0, and as buffer B 25 mM bis-tris pH 6.0. The fractions from the column were analyzed for activity using the phosphatase assay (see below) and fractions with activity were pooled.

The pooled fractions were dialyzed extensively against 10 mM Na-acatate, pH 4.5. After dialysis a minor precipitation had formed and this was removed by filtration through a Fast PES bottle top filter with a 0.22 micrometer cut-off. The phytase variant was purified by chromatography on SP Sepharose Fast Flow, approximately 50 ml in a XK26 column, using as buffer A 50 mM sodium acetate pH 4.5, and as buffer B 50 mM sodium acetate + 1 M NaCl pH 4.5. The fractions from the column were analyzed for activity using the phosphatase assay (see below) and fractions with activity were pooled. The pooled fractions were tested for phytase activity (see below) and found to be active (>200 FYT/ml).

The molecular weight, as estimated from SDS-PAGE, was approximately 50-55 kDa and the purity was > 95%.

### Determination of phosphatase activity

75 microliter phytase-containing enzyme solution is dispensed in a microtiter plate well, e. g. NUNC 269620 and 75 microliter substrate is added (for preparing the substrate, two 5 mg p-nitrophenyl phosphate tablets (Sigma, Cat.No. N-9389) are dissolved in 10 ml 0.1 M Na-acetate buffer, pH 5.5). The plate is sealed and incubated 15 min., shaken with 750 rpm at 37°C. After the incubation time 75 microliter stop reagent is added (the stop reagent is 0.1 M di-sodiumtetraborate in water) and the absorbance at 405 nm is measured in a microtiter plate spectrophotometer. One phosphatase unit is defined as the enzyme activity that releases 1 micromol phosphate/min under the given reaction conditions (buffer blind subtracted). The absorbance of 1 micromol p-nitrophenol is determined to be 56 AU (AU= absorbancy units) under assay conditions.

### Determination of phytase activity

75 microliter phytase-containing enzyme solution, appropriately diluted in 0.25M sodium acetate, 0.005% (w/v) Tween-20. pH5.5, is dispensed in a microtiter plate well, e. g. NUNC 269620, and 75 microliter substrate is added (prepared by dissolving 100mg sodium phytate from rice (Aldrich Cat.No. 274321) in 10ml 0.25M sodium acetate buffer, pH5.5). The plate is sealed and incubated 15min. shaken with 750rpm at 37°C. After incubation, 75 microliter stop reagent is added (the stop reagent being prepared by mixing 10 ml molybdate solution (10% (w/v) ammonium hepta-molybdate in 0.25% (w/v) ammonia solution), 10ml ammonium vanadate (0.24% commercial product from Bie&Berntsen, Cat.No. LAB17650), and 20ml 21.7% (w/v) nitric acid), and the absorbance at 405nm is measured in a microtiter plate spectrophotometer. The phytase activity is expressed in the unit of FYT, one FYT being the amount of enzyme that liberates 1 micromole inorganic ortho-phosphate per minute under the conditions above. An absolute value for the measured phytase activity may be obtained by reference to a standard curve prepared from appropriate dilutions of inorganic phosphate, or by reference to a standard curve made from dilutions of a phytase enzyme preparation with known activity (such standard enzyme preparation with a known activity is available on request from Novozymes A/S, Krogshoejvej 36, DK-2880 Bagsvaerd).

### Example 2: Specific activity

The specific activity of a phytase variant is determined on highly purified samples dialysed against 250 mM sodium acetate, pH 5.5. The purity is checked beforehand on an SDS poly acryl amide gel showing the presence of only one component.

The protein concentration is determined by amino acid analysis as follows: An aliquot of the sample is hydrolyzed in 6N HCI, 0.1% phenol for 16 h at 110°C in an evacuated glass tube. The resulting amino acids are quantified using an Applied Biosystems 420A amino acid analysis system operated according to the manufacturer's instructions. From the amounts of the amino acids the total mass - and thus also the concentration - of protein in the hydrolyzed aliquot can be calculated.

The phytase activity is determined in the units of FYT as described in Example 1 ("Determination of phytase activity"), and the specific activity is calculated as the phytase activity measured in FYT units per mg phytase variant enzyme protein.

### Example 3. Thermostability

An aliquot of the protein sample of E. coli phytase variant (purified as described in Example 1) was either, desalted and buffer-changed into 20 mM Na-acetate, pH 4.0 using a prepacked PD-10 column, or dialysed against 2 x 500 ml 20 mM Na-acetate, pH 4.0 at 4°C in a 2-3h step followed by an overnight step. The sample was 0.45 µm filtered and diluted with buffer to approx. 2 A280 units. The dialysis buffer was used as reference in Differential Scanning Calorimetry (DSC). The samples were degassed using vacuum suction and stirring for approx. 10 minutes.

A DSC scan was performed on a MicroCal VP-DSC at a constant scan rate of 1.5 °C/min from 20-90 °C. Data-handling was performed using the MicroCal Origin software (version 4.10), and the denaturation temperature, Td (also called the melting temperature, Tm) is defined as the temperature at the apex of the peak in the thermogram.

The results of DSC for E. coli phytase variants are summarized in the Table 2 below.

**Table 2. Comparative Thermostability of E. coli Phytases**

| **Variant** | **Modification** | **Td 1st Scan (°C)** |
|---|---|---|
| Ec-phyt01 | A116T | 61.8 |
| Ec-phyt02 | SEQ ID NO:2 | 57.6 |
| Ec-phyt06 | G52C/A99C = A | 60.1 |
| Ec-phyt07 | D31C/L177C = C | 53.4 |
| Ec-phyt08 | T141C/V200C = B | 58.7 |
| Ec-phyt09 | G52C/A99C/T141C/V200C/D31C/L177C/L410LR = A/B/C/L410LR | 66.1 |

### Example 4. Temperature profile

The temperature profile (phytase activity as a function of temperature) was determined for the *E. coli* phytase and variants in the temperature range of 20-90°C essentially as described above ("Determination of phytase activity"). However, the enzymatic reactions (100 microliter phytase-containing enzyme solution + 100 microliter substrate) were performed in PCR tubes instead of microtiter plates. After a 15 minute reaction period at desired temperature the tubes were cooled to 20°C for 20 seconds and 150 microliter of each reaction mixture was transferred to a microtiter plate. 75 microliter stop reagent was added and the absorbance at 405 nm was measured in a microtiter plate spectrophotometer. The results are summarized in Table 3 below. The numbers given for each temperature are relative activity (in %) normalized to the value at optimum.

**Table 3: Relative temperature profiles**

| **Phytase variant** | | | **Temperature (°C)** | | | | | | | | | |
|---|---|---|---|---|---|---|---|---|---|---|---|---|
| | **20** | **30** | | **40** | **50** | **60** | **65** | **70** | **75** | **80** | **85** | **90** |
| Ec-phy01 | 19 | 33 | | 53 | 81 | 100 | 86 | 23 | 14 | 12 | 10 | 7 |
| Ec-phy06 | 15 | 28 | | 45 | 70 | 89 | 100 | 89 | 21 | 14 | 11 | 9 |
| Ec-phy07 | 18 | 34 | | 54 | 82 | 100 | 83 | 18 | 13 | 11 | 10 | 7 |
| Ec-phy08 | 14 | 25 | | 38 | 61 | 82 | 100 | 38 | 13 | 10 | 9 | 7 |
| Ec-phy09 | 10 | 19 | | 33 | 54 | 69 | 95 | 99 | 100 | 16 | 10 | 6 |

### Example 5. pH profile

The pH profile was determined at 37°C in the pH range of 2.0 to 7.5 (in 0.5 pH-unit steps) as described above in the section "Determination of phytase activity", except that a buffer cocktail (50mM glycine, 50mM acetic acid and 50mM Bis-Tris was used instead of the 0.25M sodium acetate pH5.5 buffer. The results are summarized in table 5 below. The values given for each pH in the range of 2.0 - 7.5 are the relative activity in % normalized to the value at optimum.

**Table 5: Relative pH profiles at 37°C**

| **Mutation/pH** | **2** | **2,5** | **3** | **3,5** | **4** | **4,5** | **5** | **5,5** | **6** | **6,5** | **7** | **7,5** |
|---|---|---|---|---|---|---|---|---|---|---|---|---|
| Ec-phyt01 | 54 | 67 | 79 | 86 | 96 | 99 | 100 | 91 | 79 | 52 | 16 | 2 |
| Ec-phyt06 | 55 | 67 | 76 | 81 | 92 | 98 | 100 | 94 | 78 | 53 | 16 | 1 |
| Ec-phyt07 | 57 | 72 | 83 | 88 | 98 | 99 | 100 | 89 | 72 | 46 | 14 | 2 |
| Ec-phyt08 | 52 | 65 | 78 | 85 | 96 | 100 | 98 | 88 | 72 | 46 | 13 | 1 |
| Ec-phyt09 | 59 | 71 | 82 | 93 | 96 | 100 | 92 | 77 | 59 | 35 | 9 | 0 |

### Example 6: Performance in animal feed in an in vitro model

The performance in animal feed of a number of phytase variants of the invention is compared in an *in vitro* model to the performance of a reference protein such as SEQ ID NO:2. The *in vitro* model simulates gastro-intestinal conditions in a monogastric animal and correlates well with results obtained in animal trials in vivo. The version used in this example simulates the crop and stomach of a broiler. The comparison is performed as follows:
Phytase activity in the variant sample is determined as described in Example 1 under "Determination of phytase activity".

Feed pellets from a broiler feeding trial - and with maize, soybean meal and soybean oil as main constituents - are pre-incubated at 40°C and pH 4.6 for 5 minutes followed by the addition of suitable dosages of the phytases (identical dosages are used for all phytases to be tested to allow comparison), for example between 125 to 1000 phytase units FYT/kg feed, or buffer in the control samples. After 5 minutes of incubation, pepsin (3000 U/g feed) in an HCl-solution is added and in this way pH is reduced to 3. The samples are then incubated at 40°C for another 5 minutes. The reactions are stopped and phytic acid and inositol-phosphates extracted by addition of HCI to a final concentration of 0.5 M and incubation at 40°C for 2 hours, followed by one freeze-thaw cycle and 1 hour incubation at 40°C.

Phytic acid and inositol-phosphates are separated by high performance ion chromatography as described by Chen et al in Journal of Chromatography A (2003) vol. 1018, pp. 41-52 and quantified as described by Skoglund et al in J. Agric. Food Chem. (1997), vol. 45, pp. 431-436. Degradation of phytate is then calculated as the difference in inositol-6-phosphate bound phosphorous (IP6-P) between phytase-treated and non-treated samples. The relative performance of the variant is calculated as the percentage of phytate degradation by the wild type phytase.

### Example 7: Performance in an in vivo pig trial

Comparative evaluation of the effects of graded amounts of the *E. coli* wild type phytase and a variant on the faecal digestibility and excretion of phosphorus and calcium in growing pigs.

Sixty four Large White × Landrace pigs having an initial body weight of 43.55 ± 4.35 kg are used. The animals are housed in floor-pen cages in an environmentally controlled room. Each pen has a plastic-coated welded wire floor and is equipped with two water nipples and four stainless-steel individualized feeders. Room temperature was 21-22° C and humidity percentage is 50 %.

The pigs are fed a basal diet formulated to provide phosphorus (P) exclusively from vegetable origin during an adaptive period of 14 days. After that period they are allocated into 16 equal groups of 4 animals each.

They are fed for 12 days the basal diet or this diet supplemented with 1000 or 2000 U/kg of *E. coli* wild type phytase or with 500, 1000 or 2000 U/kg of the variant designated 100 having 2 additional disulfide bonds.

An indigestible tracer (chromium oxide) is added at a concentration of 0.4 % to all the diets allowing calculation of the digestibility of P and calcium (Ca). The feed is distributed *ad libitum* in mash form, under pen feed consumption control, and the animals has free access to drinking water. The digestibility of Ca is not corrected for Ca intake with the drinking water.

Faecal P, Ca and Cr concentrations are measured at the 12^{th} day of the second period. Faeces were sampled individually, in approximately the same amount at the same time of the day, during the last 3 days preceding that date. Thus, for each dietary treatment and for each criterion a total of 12 individual determinations are performed. All minerals are determined according to standard Association of Official Analytical Chemists (1990) methods using a Vista-MPX ICP-OES spectrometer. The apparent digestibility (% of the intake) of the minerals is calculated for the mentioned 3 day period.

## Claims

1. A phytase variant having at least 85% identity to SEQ ID NO:2 and comprising at least one disulfide bridge as compared to SEQ ID NO:2, wherein said at least one disulfide bridge is not among the four naturally occurring ones in positions 77/108, 133/408, 178/188, and 382/391 with the numbering as provided in SEQ ID NO:2, wherein the at least one disulfide bridge is position pair: A) 52C/99C, and wherein the phytase variant is more thermostable than the reference phytase.

2. The phytase variant of claim 1, wherein the number of established disulfide bridges is two using the following combinations of position pairs:
A+B, A+C, A+D, A+E, A+F and A+G, wherein A means 52C/99C, B means 141C/200C, C means 31C/177C, D means 91C/46C, E means 31C/176C, F means 59C/100C, and G means 162C/248C.

3. The phytase variant of claim 1, wherein the number of established disulfide bridges is three using the following combinations of position pairs:
A+B+C, A+B+D, A+B+E, A+B+F, A+B+G, A+C+D, A+C+E, A+C+F, A+C+G, A+D+E, A+D+F, A+D+G, A+E+F, A+E+G and A+F+G, wherein A means 52C/99C, B means 141C/200C, C means 31C/177C, D means 91C/46C, E means 31C/176C, F means 59C/100C, and G means 162C/248C.

4. The phytase variant of any of claims 1 to 3 further comprising a modification in at least one position selected from the following: 25, 37, 38, 75, 77, 108, 114, 123, 126, 127, 133, 137, 141, 142, 146, 157, 173, 178, 188, 204, 211, 233, 235, 253, 267, 286, 287, 317, 318, 327, 367, 382, 391, and 408.

5. The phytase variant of any of claims 1 to 4, comprising a further modification selected from the following:
A25F, W37F, P38Y, C75K, C75V, C75E, C77A, C108A, T114H, P123E, N126Y, P127L, P127V, C133A, N137E, N137V, T141R, D142R, E146R, G157R, P173Y, C178A, C188A, C204N, C204D, V211W, G233E, G235Y, Q253V, R267A, K286F, Q287Y, N317L, W318Y, T327Y, S367F, C382A, C391A, C408A, and/or from the following combinations W37F/P38Y, P123E/P127L, N126Y/P127V, G233E/G235Y, K286F/Q287Y, N317L/W318Y, W37F/P38Y/P123E/P127L, W37F/P38Y/N126Y/P127V, P173Y/N317L/W318Y, C75K/C204N, C75K/C204N/C178A/C188A,
C75K/C204N/C382A/C391A, C75K/C204N/C178A/C188A/C382A/C391A,
C75K/C204N/C77A/C108A, C75K/C204N/C133A/C108A,
A25F/C75V/T114H/N137E/T141R/E146R/G157R/C204D/V211W/Q253V/R267A/T327Y/C178A/C188A,
A25F/C75V/T114H/N137E/T141R/E146R/G157R/C204D/V211W/Q253V/R267A/T327Y/C382A/C391A,
A25F/C75V/T114H/N137E/T141R/E146R/G157R/C204D/V211W/Q253V/R267A/T327Y/C178A/C188A/C382A/C391A,
A25F/C75V/T114H/N137E/T141R/E146R/G157R/C204DA/211W/Q253V/R267A/T327Y/C77A/C108A,
A25F/C75V/T114H/N137E/T141R/E146R/G157R/C204D/V211W/Q253V/R267A/T327Y/ C133A/C408A,
A25F/C75E/T114H/N137V/G157R/C204D/V211W/Q253V/R267A/T327Y,
A25F/C75E/N137V/T141R/D142R/G157R/C204D/V211W/Q253V/T327Y,
A25F/T114H/N137V/T141R/D142R/G157R/C204D/V211W/Q253V/T327Y,
A25F/T114H/N137V/T141R/D142R/E146R/G157R/C204D/V211W/Q253V/R267A/T327Y,
A25F/C75V/T114H/N137V/T141R/E146R/G157R/C204D/V211W/Q253V/R267A/T327Y,
A25F/C75V/T114H/N137V/T141R/D142R/E146R/G157R/C204D/V211W/Q253V/T327Y,
A25F/T114H/N137V/T141R/D142R/E146R/G157R/C204D/V211W/Q253V/T327Y, A25F/C75E/T114H/N137V/T141R/D142R/G157R/C204D/V211W/Q253V/T327Y,
A25F/C75E/T114H/N137V/T141R/D142R/E146R/G157R/V211W/Q253V/R267A/T327Y,
A25F/C75E/T114H/N137E/T141R/D142R/G157R/V211W/Q253V/R267A/T327Y/L341P,
A47F/C97V/T136H/N159V/T163R/D164R/G179R/V233W/Q275V/R289A/T349Y,
A25F/C75E/T114H/N137V/T141R/D142R/E146R/G157R/C204D/V211W/Q253V/R267A/T327Y,
A25F/C75E/N137V/D142R/G157R/C204D/V211W/Q253V/R267A/T327Y,
A25F/C75E/N137E/T141R/D142R/E146R/G157R/C204D/V211W/Q253V/R267A/T327Y,
A25F/C75V/T114H/N137E/T141R/D142R/E146R/G157R/C204D/V211W/Q253V/R267A/T327Y,
A25F/C75E/T114H/N137V/T141R/G157R/C204D/V211W/Q253V/T327Y,
A25F/C75E/T114H/N137V/T141R/D142R/E146R/G157R/C204D/V211W/Q253V/T327Y,
A25F/T114H/N137E/T141R/D142R/G157R/C204D/V211W/Q253V/R267A/T327Y,
A25F/T114H/N137V/D142R/G157R/C204D/V211W/Q253V/T327Y,
A25F/C75V/T114H/N137V/D142R/G157R/C204D/V211W/Q253V/T327Y,
A25F/C75V/T114H/N137V/D142R/G157R/V211W/Q253V/T327Y, and
A25F/T114H/N137V/C204D/V211W/T327Y.

6. A method of preparing the phytase variant of any of claims 1 to 5, comprising the establishment of at least one disulfide bridge as compared to SEQ ID NO: 2, wherein said disulfide bridge is not among the four naturally occurring ones in positions 77/108, 133/408, 178/188, and 382/391 with the numbering as provided in SEQ ID NO: 2, and wherein the at least one disulfide bridge is established in position pair: A) 52C/99C.

7. The method of claim 6, wherein the mature part of the phytase of SEQ ID NO:2, SEQ ID NO:3, SEQ ID NO:4, SEQ ID NO:5, or SEQ ID NO:6 is used as a parent/backbone for producing the phytase variant.

8. A polynucleotide comprising a nucleotide sequence which encodes the phytase variant of any of claims 1 to 5.

9. A nucleic acid construct comprising the polynucleotide of claim 8 operably linked to one or more control sequences that direct the production of the polypeptide in an expression host.

10. A recombinant expression vector comprising the nucleic acid construct of claim 9.

11. A recombinant host cell comprising the nucleic acid construct of claim 9 and/or the expression vector of claim 10.

12. A method for producing the phytase variant of any of claims 1 to 5, comprising
(a) cultivating the host cell of claim 11 to produce a supernatant comprising the phytase; and
(b) recovering the phytase.

13. A composition comprising at least one phytase variant of any of claims 1 to 5.

14. The composition of claim 13 further comprising
(a) at least one fat soluble vitamin;
(b) at least one water soluble vitamin; and/or
(c) at least one trace mineral.

15. The composition of claim 14 further comprising at least one enzyme selected from the following group of enzymes: amylase, phytase, phosphatase, xylanase, galactanase, alpha-galactosidase, protease, phospholipase, and/or beta-glucanase.

16. Use of the phytase variant of any of claims 1 to 5, or the composition of any one of claims 14-15 in animal feed; in the preparation of animal feed; for improving the nutritional value of animal feed; for reducing phytate levels in animal manure; for the treatment of vegetable proteins; or for liberating phosphorous from a phytase substrate.

17. A method for producing a fermentation product comprising fermenting a carbohydrate material in the presence of the phytase variant of any of claims 1 to 5.

18. A method for producing ethanol comprising fermenting a carbohydrate material in the presence of the phytase variant of any of claims 1 to 5 and producing ethanol.

## Patentansprüche

1. Phytasevariante mit mindestens 85% Identität zu SEQ ID NO:2 und umfassend mindestens eine Disulfidbrücke verglichen zu SEQ ID NO:2, wobei sich die mindestens eine Disulfidbrücke nicht unter den natürlicherweise vorkommenden in Positionen 77/108, 133/408, 178/188 und 382/391 befindet, mit der wie in SEQ ID NO:2 bereitgestellten Nummerierung, wobei die mindestens eine Disulfidbrücke das Positionenpaar: A) 52C/99C ist, und wobei die Phytasevariante thermostabiler als die Referenz-Phytase ist.

2. Phytasevariante nach Anspruch 1, wobei die Anzahl an gebildeten Disulfidbrücken zwei beträgt, unter Verwendung der folgenden Kombinationen an Positionenpaaren:
A+B, A+C, A+D, A+E, A+F und A+G, wobei A 52C/99C bedeutet, B 141C/200C bedeutet, C 31C/177C bedeutet, D 91C/46C bedeutet, E 31C/176C bedeutet, F 59C/100C bedeutet und G 162C/248C bedeutet.

3. Phytasevariante nach Anspruch 1, wobei die Anzahl an gebildeten Disulfidbrücken drei beträgt, unter Verwendung der folgenden Kombinationen an Positionenpaaren:
A+B+C, A+B+D, A+B+E, A+B+F, A+B+G, A+C+D, A+C+E, A+C+F, A+C+G, A+D+E, A+D+F, A+D+G, A+E+F, A+E+G und A+F+G, wobei A 52C/99C bedeutet, B 141 C/200C bedeutet, C 31C/177C bedeutet, D 91C/46C bedeutet, E 31C/176C bedeutet, F 59C/100C bedeutet und G 162C/248C bedeutet.

4. Phytasevariante nach einem beliebigen der Ansprüche 1 bis 3, die des Weiteren eine Modifikation in mindestens einer Position ausgewählt aus den folgenden umfasst: 25, 37, 38, 75, 77, 108, 114, 123, 126, 127, 133, 137, 141, 142, 146, 157, 173, 178, 188, 204, 211, 233, 235, 253, 267, 286, 287, 317, 318, 327, 367, 382, 391 und 408.

5. Phytasevariante nach einem beliebigen der Ansprüche 1 bis 4, die des Weiteren eine weitere Modifikation ausgewählt aus den folgenden umfasst:
A25F, W37F, P38Y, C75K, C75V, C75E, C77A, C108A, T114H, P123E, N126Y, P127L, P127V, C133A, N137E, N137V, T141R, D142R, E146R, G157R, P173Y, C178A, C188A, C204N, C204D, V211W, G233E, G235Y, Q253V, R267A, K286F, Q287Y, N317L, W318Y, T327Y, S367F, C382A, C391A, C408A, und/oder aus den folgenden Kombinationen W37F/P38Y, P123E/P127L, N126Y/P127V, G233E/G235Y, K286F/Q287Y, N317L/W318Y, W37F/P38Y/P123E/P127L, W37F/P38Y/N126Y/P127V, P173Y/N317L1W318Y, C75K/C204N, C75K/C204N/C178A/C188A,
C75K/C204N/C382A/C391A, C75K/C204N/C178A/C188A/C382A/C391A,
C75K/C204N/C77A/C108A, C75K/C204N/C133A/C108A,
A25F/C75V/T114H/N137E/T141R/E146R/G157R/C204D/V211W/Q253V/R267A/T327Y/C178A/C188A,
A25F/C75V/T114H/N137E/T141R/E146R/G157R/C204DA/211W/Q253V/R267A/T327Y/ C382A/C391A,
A25F/C75V/T114H/N137E/T141R/E146R/G157R/C204D/V211W/Q253V/R267A/T327Y/ C178A/C188A/C382A/C391A,
A25F/C75V/T114H/N137E/T141R/E146R/G157R/C204DA/211W/Q253V/R267A/T327Y/C77A/C108A,
A25F/C75V/T114H/N137E/T141R/E146R/G157R/C204D/V211W/Q253V/R267A/T327Y/ C133A/C408A,
A25F/C75E/T114H/N137V/G157R/C204D/V211W/Q253V/R267A/T327Y,
A25F/C75E/N137V/T141R/D142R/G157R/C204D/V211W/Q253V/T327Y,
A25F/T114H/N137V/T141R/D142R/G157R/C204D/V211W/Q253V/T327Y,
A25F/T114H/N137V/T141R/D142R/E146R/G157R/C204D/V211W/Q253V/R267A/T327Y,
A25F/C75V/T114H/N137V/T141R/E146R/G157R/C204D/V211W/Q253V/R267A/T327Y,
A25F/C75V/T114H/N137V/T141R/D142R/E146R/G157R/C204D/V211W/Q253V/T327Y,
A25F/T114H/N137V/T141R/D142R/E146R/G157R/C204D/V211W/Q253V/T327Y,
A25F/C75E/T114H/N137V/T141R/D142R/G157R/C204D/V211W/Q253V/T327Y,
A25F/C75E/T114H/N137V/T141R/D142R/E146R/G157R/V211W/Q253V/R267A/T327Y,
A25F/C75E/T114H/N137E/T141R/D142R/G157R/V211W/Q253V/R267A/T327Y/L341P,
A47F/C97V/T136H/N159V/T163R/D164R/G179RA/233W/Q275V/R289A/T349Y,
A25F/C75E/T114H/N137V/T141R/D142R/E146R/G157R/C204DA/211W/Q253V/R267A/T327Y,
A25F/C75E/N137V/D142R/G157R/C204D/V211W/Q253V/R267A/T327Y,
A25F/C75E/N137E/T141R/D142R/E146R/G157R/C204D/V211W/Q253V/R267A/T327Y,
A25F/C75V/T114H/N137E/T141R/D142R/E146R/G157R/C204DA/211W/Q253V/R267A/T327Y,
A25F/C75E/T114H/N137V/T141R/G157R/C204D/V211W/Q253V/T327Y,
A25F/C75E/T114H/N137V/T141R/D142R/E146R/G157R/C204D/V211W/Q253V/T327Y,
A25F/T114H/N137E/T141R/D142R/G157R/C204DA/211W/Q253V/R267A/T327Y,
A25F/T114H/N137V/D142R/G157R/C204D/V211W/Q253V/T327Y,
A25F/C75V/T114H/N137V/D142R/G157R/C204D/V211W/Q253V/T327Y,
A25F/C75V/T114H/N137V/D142R/G157RA/211W/Q253V/T327Y und
A25F/T114H/N137V/C204D/V211W/T327Y.

6. Verfahren zum Herstellen der Phytasevariante gemäß einem beliebigen der Ansprüche 1 bis 5, umfassend das Bilden von mindestens einer Disulfidbrücke verglichen zu SEQ ID NO: 2, wobei sich die mindestens eine Disulfidbrücke nicht unter den natürlicherweise vorkommenden in Positionen 77/108, 133/408, 178/188 und 382/391 befindet, mit der wie in SEQ ID NO: 2 bereitgestellten Nummerierung, und wobei die mindestens eine Disulfidbrücke im Positionenpaar: A) 52C/99C gebildet ist.

7. Verfahren nach Anspruch 6, wobei der reife Teil der Phytase von SEQ ID NO:2, SEQ ID NO:3, SEQ ID NO:4, SEQ ID NO:5 oder SEQ ID NO:6 als ein Parentales/Rückgrat zum Herstellen der Phytasevariante verwendet wird.

8. Polynukleotid, das eine Nukleotidsequenz umfasst, die die Phytasevariante gemäß einem beliebigen der Ansprüche 1 bis 5 kodiert.

9. Nukleinsäurekonstrukt, das das Polynukleotid gemäß Anspruch 8 umfasst, funktionsfähig verknüpft mit einer oder mehreren Kontrollsequenzen, die die Herstellung des Polypeptids in einem Expressionswirt steuert/steuern.

10. Rekombinanter Expressionsvektor, der das Nukleinsäurekonstrukt gemäß Anspruch 9 umfasst.

11. Rekombinante Wirtszelle, die das Nukleinsäurekonstrukt gemäß Anspruch 9 und/oder den Expressionsvektor gemäß Anspruch 10 umfasst.

12. Verfahren zum Herstellen der Phytasevariante gemäß einem beliebigen der Ansprüche 1 bis 5, umfassend
(a) Kultivieren der Wirtszelle gemäß Anspruch 11, um einen Überstand herzustellen, der die Phytase umfasst; und
(b) Gewinnen der Phytase.

13. Zusammensetzung, die mindestens eine Phytasevariante gemäß einem beliebigen der Ansprüche 1 bis 5 umfasst.

14. Zusammensetzung nach Anspruch 13, die des Weiteren umfasst
(a) mindestens ein fettlösliches Vitamin;
(b) mindestens ein wasserlösliches Vitamin; und/oder
(c) mindestens ein Spurenmineral.

15. Zusammensetzung nach Anspruch 14, die des Weiteren mindestens ein Enzym umfasst, das aus der folgenden Gruppe an Enzymen ausgewählt ist: Amylase, Phytase, Phosphatase, Xylanase, Galactanase, alpha-Galactosidase, Protease, Phospholipase, und/oder beta-Glucanase.

16. Verwendung der Phytasevariante gemäß einem beliebigen der Ansprüche 1 bis 5, oder der Zusammensetzung gemäß einem beliebigen der Ansprüche 14-15 in Tierfutter; in der Herstellung von Tierfutter; zum Verbessern des Nährwerts von Tierfutter; zum Verringern von Phytatspiegeln in Tierdung; zur Behandlung von pflanzlichen Proteinen; oder zum Freisetzen von Phosphor aus einem Phytasesubstrat.

17. Verfahren zum Herstellen eines Fermentationsprodukts, das Fermentieren eines Kohlenhydratmaterials in der Gegenwart der Phytasevariante gemäß einem beliebigen der Ansprüche 1 bis 5 umfasst.

18. Verfahren zum Herstellen von Ethanol, das Fermentieren eines Kohlenhydratmaterials in der Gegenwart der Phytasevariante gemäß einem beliebigen der Ansprüche 1 bis 5 und Herstellen von Ethanol umfasst.

## Revendications

1. Variant de phytase présentant une identité d'au moins 85 % avec SEQ ID No : 2 et comprenant au moins un pont disulfure comparativement à SEQ ID No : 2, dans lequel ledit au moins pont disulfure ne fait pas partie des quatre ponts disulfure naturels aux positions 77/108, 133/408, 178/188, et 382/391 selon la numérotation fournie dans SEQ ID No : 2, où ledit au moins pont disulfure est établi à la paire de positions : A) 52C/99C, et le variant de phytase est plus thermostable que la phytase de référence.

2. Variant de phytase selon la revendication 1, dans lequel le nombre de ponts disulfure établis est de deux en utilisant les combinaisons de paires de positions suivantes :
A+B, A+C, A+D, A+E, A+F et A+G, où A signifie 52C/99C, B signifie 141C/200C, C signifie 31C/177C, D signifie 91C/46C, E signifie 31C/176C, F signifie 59C/100C, et G signifie 162C/248C.

3. Variant de phytase selon la revendication 1, dans lequel le nombre de ponts disulfure établis est de trois en utilisant les combinaisons de paires de positions suivantes :
A+B+C, A+B+D, A+B+E, A+B+F, A+B+G, A+C+D, A+C+E, A+C+F, A+C+G, A+D+E, A+D+F, A+D+G, A+E+F, A+E+G, et A+F+G, où A signifie 52C/99C, B signifie 141C/200C, C signifie 31C/177C, D signifie 91C/46C, E signifie 31C/176C, F signifie 59C/100C, et G signifie 162C/248C.

4. Variant de phytase selon l'une quelconque des revendications 1 à 3 comprenant en outre une modification à au moins une position choisie parmi les suivantes : 25, 37, 38, 75, 77, 108, 114, 123, 126, 127, 133, 137, 141, 142, 146, 157, 173, 178, 188, 204, 211, 233, 235, 253, 267, 286, 287, 317, 318, 327, 367, 382, 391, et 408.

5. Variant de phytase selon l'une quelconque des revendications 1 à 4, comprenant une modification supplémentaire choisie parmi les suivantes :
A25F, W37F, P38Y, C75K, C75V, C75E, C77A, C108A, T114H, P123E, N126Y, P127L, P127V, C133A, N137E, N137V, T141R, D142R, E148R, G157R, P173Y, C178A, C188A, C204N, C204D, V211W. G233E, G235Y, Q253V, R267A, K286F, Q287Y. N317L, W318Y, T327Y, S367F, C382A, C391A, C408A,
et/ou parmi les combinaisons suivantes :
W37F/P38Y, P123E/P127L, N126Y/P127V, G233E/G235Y,
K286F/Q287Y, N317L/W318Y. W37F/P38Y/P123E/P127L, W37F/P38Y/M126Y/P127V P173Y/N317L/W318Y, C75K/C204N C75K/C2G4N/C178A/C188A,
C75K/C204N/C382A/C391A, C75K/C204N/C178A/C188A/C382A/C391A,
C75K/C204N/C77A/C108A, C75K/C204N/C133A/C108A,
A25F/C75V/T114H/N137E/T141R/E146R/G157R/C204D/V211W/Q253V/R267A/T327Y/C178A/C188A,
A25F/G75V/T114H/N137E/T141R/E146R/G157R/C204D/V211W/Q253V/R267A/T327Y/C382A/C391A,
A25F/C75V/T114H/N137E/T141R/E146R/G157R/C204D/V211W/Q253V/R267A/T327Y/C178A/C188A/C382A/C391A,
A25F/C75V/T114H/N137E/T141R/E146R/G157R/C204D/V211W/Q253V/R267A/T327Y/C77A/C108A.
A25F/C75V/T114H/N137E/T141R/E146R/G157R/C204D/V211W/Q253V/R267A/T327Y/C133A/C408A,
A25F/C75E/T114H/N137V/G157R/C204D/V211W/Q253V/R267A/T327Y,
A25F/C75E/N137V/T141R/D142R/G157R/C204D/V211W/Q253V/T327Y,
A25F/T114H/N137V/T141R/D142R/G157R/C204D/V211W/Q253V/T327Y,
A25F/T114H/N137V/T141R/D142R/E146R/G157R/C204D/V211W/Q253V/R267A/T327Y,
A25F/C75V/T114H/N137V/T141R/E146R/G157R/C204D/V211W/Q253V/R267A/T327Y,
A25F/C75V/T114H/N137V/T141R/D142R/E146R/G157R/C204D/V211W/Q253V/T327Y,
A25F/T114H/N137V/T141R/D142R/E146R/G157R/C204D/V211W/Q253V/T327Y,
A25F/C75E/T114H/N137V/T141R/D142R/G157R/C204D/V211W/Q253V/T327Y,
A25F/C75E/T114H/N137V/T141R/D142R/E146R/G157R/V211W/Q253V/R267A/T327Y,
A25F/G75E/T114H/N137E/T141R/D142R/G157R/V211W/Q253V/R267A/T327Y/L341P,
A47F/C97V/T136H/N159V/T163R/D164R/G179R/V233W/Q275V/R289A/T349Y,
A25F/C75E/T114H/N137V/T141R/D142R/E146R/G157R/C204D/V211W/Q253V/R267A/T327Y,
A25F/C75E/N137V/D142R/G157R/C204D/V211W/Q253V/R267A/T327Y,
A25F/C75E/N137E/T141R/D142R/E146R/G157R/C204D/V211W/Q253V/R267A/T327Y,
A25F/C75V/T114H/N137E/T141R/D142R/E146R/G157R/C204D/V211W/Q253V/R267A/T327Y,
A25F/C75E/T114H/N137V/T141R/G157R/C204D/V211W/Q253V/T327Y.
A25F/C75E/T114H/N137V/T141R/D142R/E146R/G157R/C204D/V211W/Q253V/T327Y,
A25F/T114H/N137E/T141R/D142R/G157R/C204D/V211W/Q253V/R267A/T327Y
A25F/T114H/N137V/D142R/G157R/C204D/V211W/Q253V/T327Y,
A25F/C75V/T114H/N137V/D142R/G157R/C204D/V211W/Q253V/T327Y,
A25F/C75V/T114H/N137V/D142R/G157R/V211W/Q253V/T327Y, et
A25F/T114H/N137V/C204D/V211W/T327Y.

6. Méthode de préparation du variant de phytase selon l'une quelconque des revendications 1 à 5, comprenant l'établissement d'au moins un pont disulfure comparativement à SEQ ID No : 2, où ledit au moins pont disulfure ne fait pas partie des quatre ponts disulfure naturels aux positions 77/108, 133/408, 178/188, et 382/391 selon la numérotation fournie dans SEQ ID No : 2, et où ledit au moins pont disulfure est établi à la paire de positions : A) 52C/99C.

7. Méthode selon la revendication 6, dans laquelle la partie mature de la phytase de SEQ ID No : 2, SEQ ID No : 3, SEQ ID No : 4, SEQ ID No : 5, ou SEQ ID No : 6 est utilisée comme parent/squelette pour produire le variant de phytase.

8. Polynucléotide comprenant une séquence de nucléotides qui code pour le variant de phytase selon l'une quelconque des revendications 1 à 5.

9. Construction d'acide nucléique comprenant le polynucléotide selon la revendication 8 fonctionnellement liée à une ou plusieurs séquences de contrôle qui dirigent la production du polypeptide chez un hôte d'expression.

10. Vecteur d'expression recombiné comprenant la construction d'acide nucléique selon la revendication 9.

11. Cellule hôte recombinée comprenant la construction d'acide nucléique selon la revendication 9 et/ou le vecteur d'expression selon la revendication 10.

12. Méthode de production du variant de phytase selon l'une quelconque des revendications 1 à 5, comprenant
(a) la culture de la cellule hôte selon la revendication 11 pour produire un surnageant contenant la phytase ; et
(b) la récupération de la phytase.

13. Composition comprenant au moins un variant de phytase selon l'une quelconque des revendications 1 à 5.

14. Composition selon la revendication 13 comprenant en outre
(a) au moins une vitamine liposoluble ;
(b) au moins une vitamine hydrosoluble ; et/ou
(c) au moins un minéral à l'état de traces.

15. Composition selon la revendication 14 comprenant en outre au moins une enzyme choisie dans le groupe d'enzymes suivant : amylase, phytase, phosphatase, xylanase, galactanase, alpha-galactosidase, protéase, phospholipase, et/ou bêta-glucanase.

16. Utilisation du variant de phytase selon l'une quelconque des revendications 1 à 5, ou de la composition selon l'une quelconque des revendications 14 à 15 dans l'alimentation animale ; dans la préparation d'un aliment pour animaux ; pour améliorer la valeur nutritionnelle de l'alimentation animale ; pour réduire les taux de phytase dans le fumier animal ; pour traiter les protéines végétales ; ou pour libérer le phosphore d'un substrat de phytase.

17. Méthode de production d'un produit de fermentation comprenant la fermentation d'une substance glucidique en présence d'un variant de phytase selon l'une quelconque des revendications 1 à 5.

18. Méthode de production d'éthanol comprenant la fermentation d'une substance glucidique en présence du variant de phytase selon l'une quelconque des revendications 1 à 5 et la production d'éthanol.
